(19) Europäisches Patentamt
European Patent Office
Office européen des brevets

(11) Veröffentlichungsnummer : **0 447 828 B1**

(12) **EUROPÄISCHE PATENTSCHRIFT**

(45) Veröffentlichungstag der Patentschrift : **04.05.94 Patentblatt 94/18**

(51) Int. Cl.$^5$ : **C07C 211/40,** C07C 211/62, C07C 49/657, C07C 209/24, A01N 33/04, A01N 33/12

(21) Anmeldenummer : **91102596.3**

(22) Anmeldetag : **22.02.91**

# (54) 4-(4-tert.-Butylphenyl)cyclohexylamine und diese enthaltende Fungizide.

(30) Priorität : **06.03.90 DE 4006937**

(43) Veröffentlichungstag der Anmeldung : **25.09.91 Patentblatt 91/39**

(45) Bekanntmachung des Hinweises auf die Patenterteilung : **04.05.94 Patentblatt 94/18**

(84) Benannte Vertragsstaaten : **AT BE CH DE DK ES FR GB GR IT LI NL SE**

(56) Entgegenhaltungen :
**EP-A- 0 258 853**
**EP-A- 0 259 977**
**EP-A- 0 269 041**
**US-A- 2 967 125**

(73) Patentinhaber : **BASF Aktiengesellschaft**
**Carl-Bosch-Strasse 38**
**D-67063 Ludwigshafen (DE)**

(72) Erfinder : **Zipperer, Bernhard, Dr.**
**Am Herrgottsacker 6**
**W-6716 Dirmstein (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 4-(4-tert.-Butylphenyl)cyclohexylamine, Säureadditionssalze und Quartärsalze derselben, Verfahren und Zwischenprodukte zu ihrer Herstellung, ihre Verwendung als Fungizide, fungizide Mittel sowie Verfahren zur Bekämpfung von Schadpilzen mit diesen Wirkstoffen.

Die Verbindung trans-4-tert.-Butyl-N-benzyl-cyclohexylamin ist bekannt (J. Org. Chem. 48 (1983) 3412-3422), jedoch ist über ihre fungizide Wirkung nichts bekannt.

4-(Cyclohexylmethyl)cyclohexylamin und sein N,N-Dimethylderivat sind als Fungizide bekannt (US-3 981 766). Ihre fungizide Wirkung ist jedoch unbefriedigend.

Das 1-[4-(4-tert.-Butylphenyl)-cyclohexyl]-2,6-dimethylmorpholin ist als Fungizid beschrieben (EP 259 977). Seine Wirksamkeit ist jedoch in manchen Anwendungsgebieten, insbesondere bei niedrigen Aufwandmengen und Konzentrationen, schlecht.

Fungizide Cyclohexylamine sind aus DE 36 40 247 bekannt. Ihre fungizide Wirkung ist gut; ihre Pflanzenverträglichkeit ist jedoch, insbesondere bei höheren Aufwandmengen, unbefriedigend.

Es wurde nun gefunden, daß 4-(4-tert.-Butylphenyl)cyclohexylamine und ihre quaternären Ammoniumsalze der Formeln

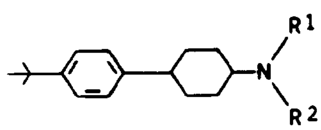

und

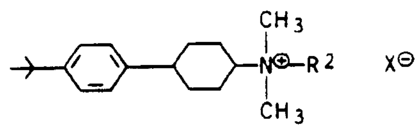

1 2

in der

$R^1$

Wasserstoff, $C_1$-$C_6$-Alkyl, $C_3$-$C_6$-Alkenyl und

$R^2$

$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein- bis dreifach substituiertes Phenyl, unsubstituiertes oder ggf. ein- bis dreifach substituiertes Phenyl ($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können, mit der Maßgabe, daß nicht $R^1$ und $R^2$ gleichzeitig $C_1$-$C_4$-Alkyl bedeuten,

$X^\ominus$

ein pflanzenverträgliches Säureanion bedeutet

und ihre pflanzenverträglichen Säureadditionssalze, eine starke fungizide Wirkung und eine überraschend gute Pflanzenverträglichkeit aufweisen.

$R^1$

bedeutet beispielsweise Wasserstoff, geradkettiges oder verzweigtes $C_1$-$C_6$-Alkyl insbesondere $C_1$-$C_4$-Alkyl wie Methyl, Ethyl, n-Propyl, iso-Propyl, n-Butyl, iso-Butyl, sec.-Butyl, tert.-Butyl, n-Pentyl, iso-Pentyl, tert.-Pentyl, n-Hexyl, iso-Hexyl, neo-Hexyl oder geradkettiges oder verzweigtes $C_2$-$C_6$-Alkenyl wie 2-Propen-1-yl, cis- und trans-2-Buten-1-yl, 2-Methyl-2-propen-1-yl, 3-Buten-2-yl, 3-Methyl-2-buten-1-yl.

$R^2$

bedeutet beispielsweise geradkettiges oder verzweigtes $C_1$-$C_{12}$-Alkyl insbesondere $C_1$-$C_4$-Alkyl wie Methyl, Ethyl, n-Propyl, iso-Propyl, n-Butyl, iso-Butyl, sec.-Butyl, tert.-Butyl, n-Pentyl, iso-Pentyl, neo-Pentyl, n-Hexyl, neo-Hexyl, n-Heptyl, n-Octyl, 2-Ethylhexyl, n-Nonyl, n-Decyl, n-Undecyl, n-Dodecyl $C_1$-$C_{12}$-Halogenalkyl wie Chlormethyl, Brommethyl, Jodmethyl, 2-Chlorethyl, 4-Chlor-1-butyl, 3-Chlor-1-butyl, 3-Chlor-2-methyl-1-propyl, 5-Chlor-1-pentyl, 6-Chlor-1-hexyl; $C_1$-$C_{12}$-Hydroxyalkyl wie 2-Hydroxyethyl, 2-Hydroxy-1-propyl, 6-Hydroxy-1-hexyl, 8-Hydroxy-1-octyl, 10-Hydroxy-1-decyl; $C_3$-$C_{12}$-Cycloalkyl wie Cyclopropyl, Cyclobutyl, Cyclopentyl, cyclohexyl, Cycloheptyl, Cyclodecyl, Cyclododecyl, $C_4$-$C_{12}$-Alkylcycloalkyl wie Methylcyclopropyl, Methylcyclopentyl, 2-, 3- oder 4-Methylcyclohexyl, 2-, 3- oder 4-Ethylcyclohexyl, 4-iso-Propylcyclohexyl, 4-tert.-Butylcyclohexyl, 2,2-, 3,3- oder 4,4-Dimethylcyclohexyl, 2,6-Dimethylcyclohexyl, 3, 3, 5-Tetramethylcyclohexyl; $C_7$-$C_{12}$-Bicycloalkyl wie Bicyclo[2.2.1]-hept-2-yl, 1,7,7-Trimethylbicyclo[2.2.1]hept-2-yl, Bicyclo[4.4.0]dec-2-yl, Bicyclo[4.4.0]dec-3-yl; Phenyl, $C_1$-$C_4$-Alkylphenyl, Mono-, Di- oder Trimethylphenyl, Ethylphenyl, Isopropylphenyl, tert.-Butylphenyl, $C_1$-$C_4$-Alkoxyphenyl, Mono-, Di- oder Trimethoxyphenyl, n- oder tert.-Butoxyphenyl, $C_1$-$C_4$-Halogenalkylphenyl, $C_1$-$C_4$-Halogenalkoxyphenyl, Trifluormethylphenyl,

Difluormethoxyphenyl, Trifluormethoxyphenyl, Tetrafluorethoxyphenyl, Cyanophenyl, Nitrophenyl, Mono-, Di- oder Trichlorphenyl, Mono-, Di- oder Trifluorphenyl, Chlorfluorphenyl, Bromphenyl, Aryl($C_1$-$C_3$)-alkyl wie Benzyl, $C_1$-$C_4$-Alkylbenzyl, Mono-, Di-oder Trimethylbenzyl, tert.-Butylbenzyl, Halogenbenzyl, Fluorbenzyl, Mono-, Di- oder Trichlorbenzyl, $C_1$-$C_4$-Alkoxybenzyl, Mono-, Di- oder Trimethoxybenzyl, Cyanbenzyl, Nitrobenzyl, 2-Phenylethyl, 2-(Methoxyphenyl)ethyl, 2-(Chlorphenyl)ethyl, 2-(Fluorphenyl)ethyl, 2-(tert.-Butylphenyl)ethyl, 3-Phenylpropyl, 3-(Chlorphenyl)propyl, 3-(Fluorphenyl)propyl.

$X^{\ominus}$

bedeutet ein anorganisches oder organisches Säureanion wie beispielsweise Chlorid, Bromid, Jodid, Sulfat, Hydrogensulfat, Phosphat, Hydrogenphosphat, Dihydrogenphosphat, Nitrat, Tetrafluoroborat; Formiat, Acetat, Oxalat, Methansulfonat, Benzolsulfonat, p-Toluolsulfonat, Dodecylbenzolsulfonat.

Säuren für die Herstellung der Säureadditionssalze sind z.B. Mineralsäuren, Salzsäure, Schwefelsäure, Salpetersäure, Ameisensäure, Alkylcarbonsäuren wie Essigsäure, Propionsäure, Oxalsäure, Sulfonsäuren wie Methansulfonsäure, Benzolsulfonsäure, p-Toluolsulfonsäure, Dodecylbenzolsulfonsäure.

Die neuen Verbindungen der Formeln 1 und 2 können in zwei diastereomeren Formen, nämlich als cis-1,4- und als trans-1,4-disubstituiertes Cyclohexan vorliegen. Sie werden je nach Art ihrer Herstellung entweder als einheitliche Diastereomere oder als Diastereomerengemische erhalten. Aus letzteren können gegebenenfalls einheitliche Diastereomere durch allgemein bekannte Trennmethoden, beispielsweise durch Chromatographie oder fraktionierende Kristallisation gewonnen werden. Die vorliegende Erfindung umfaßt sowohl die einheitlichen Diastereomeren als auch ihre Gemische.

Bei einigen der erfindungsgemäßen Reste $R^2$ können zusätzlich zur oben genannten cis-/trans-Isomerie weitere Isomere auftreten. Bei diesen Isomeren kann es sich je nach Art des Restes $R^2$ um Enantiomere oder um Diastereomere handeln. Auch hier können die diastereomeren Verbindungen nach den üblichen Methoden, z.B. durch Chromatographie oder Kristallisation, getrennt werden. Alle isomeren Verbindungen und deren Gemische untereinander werden von der vorliegenden Erfindung umfaßt. Für die Anwendung der neuen Amine als Fungizid sind sowohl die einheitlichen Diastereomeren bzw. Enantiomeren wie auch deren Gemische geeignet. Bevorzugt werden die Gemische verwendet.

Die Erfindung umfaßt auch Verfahren zur Herstellung der neuen Amine der Formel 1.

a)

Man kann diese Amine z.B. dadurch herstellen, daß man ein Amin der Formel

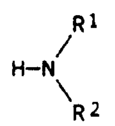

3

in der

$R^1$

Wasserstoff, $C_1$-$C_6$-Alkyl, $C_3$-$C_6$-Alkenyl und

$R^2$

$C_1$-$C_{12}$-Alkyl, mit der Maßgabe, daß nicht $R^1$ und $R^2$ gleichzeitig $C_1$-$C_4$-Alkyl bedeuten sollen, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein- bis dreifach substituiertes Phenyl, unsubstituiertes oder ggf. ein- bis dreifach substituiertes Phenyl($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können, mit 4-(4-tert.-Butylphenyl)cyclohexanon umsetzt und das als Reaktionsprodukt entstandene Imin (im Fall von $R^1$ = Wasserstoff) oder Enamin (im Fall von $R^1$ verschieden von Wasserstoff) direkt oder nach Isolierung mit einem Reduktionsmittel zu einem Amin der Formel 1 reduziert.

Es ist vorteilhaft, das bei der Umsetzung eines Amins der Formel 3, in der $R^1$ und $R^2$ die oben angegebene Bedeutung haben, mit 4-(4-tert.-Butylphenyl)cyclohexanon freiwerdende Reaktionswasser aus dem Reaktionsgemisch zu entfernen. Dies kann beispielsweise durch Zugabe eines wasserentziehenden Mittels oder durch azeotrope Destillation erfolgen. Als wasserentziehende Mittel eignen sich z.B. hydratwasserfreie oder -arme Salze wie Natriumsulfat, Magnesiumsulfat, Zinksulfat, Calciumchlorid oder Molekularsiebe. Die Reaktion wird gegebenenfalls in einem inerten organischen Lösungsmittel und gegebenenfalls in Gegenwart einer katalytischen Menge Säure durchgeführt. Als Lösungsmittel kommen Kohlenwasserstoffe wie Cyclohexan, Benzol, Toluol, Xylole, chlorierte Kohlenwasserstoffe wie Dichlormethan, 1,2-Dichlorethan oder Ether wie Diethylether, Methyl-tert.-butylether, Tetrahydrofuran, Dioxan in Frage. Als Säuren eignen sich beispielsweise Mineralsäuren wie Schwefelsäure oder Phosphorsäure, Sulfonsäuren wie Methansulfonsäure, Benzolsulfonsäure, p-Toluolsulfonsäure. Die erforderliche Säuremenge liegt z.B. bei 0 bis 10 Mol-% bezüglich eingesetztem Amin der Formel 3, bevorzugt bei 0 bis 1 Mol-%. Die Reaktion kann bei Raumtemperatur oder bei erhöhter Temperatur z.B. bis zur Siedetemperatur des jeweiligen Lösungsmittels durchgeführt werden. Falls die erforderliche Reaktionstemperatur unter Normaldruck nicht erreichbar ist oder die Umsetzung nur langsam abläuft, kann im Autoklaven (mit oder ohne Lösungsmittel) unter dem Eigendruck der Reaktionsmischung bei höherer Temperatur gearbeitet werden. Wird das entstehende Reaktionswasser azeotrop aus dem Reaktionsgemisch abdestilliert, so führt man die Umsetzung bei der Siedetemperatur des betreffenden Lösungsmittels durch. Als Lösungsmittel hierfür werden aromatische Kohlenwasserstoffe wie Benzol, Toluol oder Xylole bevorzugt.

Die bei der Umsetzung eines Amins der Formel 3, in der $R^1$ und $R^2$ die oben genannte Bedeutung haben, mit 4-(4-tert.-Butylphenyl)cyclohexanon entstandene ungesättigte Stickstoffverbindung (Imin oder Enamin) kann nach bekannten Verfahren zu einem Amin der Formel 1 reduziert werden. Als bevorzugte Reduktionsmittel seien genannt: Wasserstoff, Ameisensäure, Komplexe Hydride wie Natriumborhydrid, Natriumcyanborhydrid. Besonders bevorzugt wird Wasserstoff in Gegenwart eines metallischen Katalysators verwendet. Als Katalysatoren eignen sich beispielsweise feinverteilte Metalle wie Raney-Nickel oder Raney-Cobalt sowie Edelmetalle wie Palladium oder Platin mit oder ohne festen Trägerstoff. Die Hydrierung mit Wasserstoff kann drucklos oder unter Druck erfolgen (vgl. z.B. Houben-Weyl, Methoden der Organischen Chemie, Band 11/1, S. 602 ff, G. Thieme Verlag, Stuttgart 1957). Es ist zuweilen vorteilhaft, die Amine der Formel 1 in einem Schritt aus Aminen der Formel 3 und 4-(4-tert.-Butylphenyl)cyclohexanon herzustellen.

Ein bekanntes Verfahren hierzu, bei dem Ameisensäure als Reduktionsmittel dient, ist die reduktive Aminierung nach Leuckardt-Wallach (vgl. z.B. Houben-Weyl, Methoden der Organischen Chemie, Band 11/1, S. 648 ff, G. Thieme Verlag, Stuttgart 1957). Ein weiteres, insbesondere zur Synthese von Labormengen vorteilhaftes Verfahren verwendet Natriumcyanborhydrid als Reduktionsmittel (vgl. z.B. C.F. Lane, Synthesis 1975, 135). Als besonders zweckmäßig hat sich die Kombination Natriumcyanborhydrid/wasserfreies Zinkchlorid erwiesen (vgl. S. Kim et.al, J. Org. Chem. 50 (1985) 1927).

Bei diesem Verfahren kann das Molverhältnis $NaBH_3CN/ZnCl_2$ beispielsweise 1:2 bis 1:0,5 betragen; bevorzugt wird ein Molverhältnis 1:0,5. Bezüglich der eingesetzten Menge $NaBH_3CN$ kann entweder die Aminkomponente 3 oder die Ketonkomponente äquimolar eingesetzt werden; ein überschuß der jeweils anderen Komponente ist zuweilen zur Beschleunigung oder Vervollständigung der Umsetzung von Vorteil. Die Reaktion wird bevorzugt in einem niederen Alkohol wie Methanol, Ethanol, n-Propanol, iso-Propanol, besonders bevorzugt in Methanol als Lösungsmittel durchgeführt, wobei die Temperatur zwischen 0°C und der Siedetemperatur des jeweiligen Lösungsmittels gewählt werden kann. Bevorzugt wird bei Zimmertemperatur gearbeitet.

Das als Ausgangsstoff verwendete 4-(4-tert.-Butylphenyl)cyclohexanon ist neu. Es kann aus dem bekannten, im Handel erhältlichen 4-Phenylcyclohexanon durch Friedel-Crafts-Alkylierung des Phenylrings hergestellt werden. Als Alkylierungsmittel können z.B. tert.-Butylchlorid oder -bromid, tert.-Butanol oder 2-Methylpropen verwendet werden (vgl. z.B. Houben-Weyl, Methoden der organischen Chemie, Bd. 5/2b, S, 154 f., S. 179 f., G. Thieme Verlag, Stuttgart 1981). Bevorzugt kommt 2-Methylpropen in Gegenwart einer Mineralsäure, wie beispielsweise Schwefelsäure oder Phosphorsäure oder in Gegenwart einer Lewis-Säure wie Aluminiumtrichlorid, Eisen(III)chlorid, Bortrifluorid zum Einsatz. Als Lösungsmittel eignen sich vor allem chlorierte Kohlenwasserstoffe, insbesondere Dichlormethan, Tetrachlormethan, 1,2-Dichlorethan. Die tert.-Butylierung des 4-Phenyl-cyclohexanons kann bei Raumtemperatur oder darunter, vorzugsweise bei 0-20°C vorgenommen werden.

Die Amine der Formel 3, in der $R^1$ und $R^2$ die oben genannten Bedeutungen haben, sind bekannte Verbindungen und, soweit nicht im Handel erhältlich, nach bekannten Verfahren herstellbar.

Bei nach dem oben beschriebenen Verfahren hergestellten Aminen der Formel 1, in der $R^1$ und $R^2$ die oben genannten Bedeutungen haben, handelt es sich in der Regel um Gemische der beiden möglichen Stereoisomeren mit cis- bzw. trans-1,4-disubstituiertem Cyclohexanring. Diese cis-/trans-Gemische können gegebenenfalls durch bekannte Trennmethoden, beispielsweise durch Chromatographie oder durch fraktionierende Kristallisation in ihre Bestandteile getrennt werden.

b)

Ein weiteres Verfahren erlaubt die gezielte Herstellung von cis-oder trans-4-(4-tert.-Butylphenyl)cyclohexylaminen der Formel 1, in der

$R^1$ Wasserstoff und

$R^2$

$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein- bis dreifach substituiertes Phenyl, unsubstituiertes oder ggf. ein- bis dreifach substituiertes Phenyl($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können.

Dieses Verfahren ist dadurch gekennzeichnet, daß man zunächst 4-(4-tert.-Butylphenyl)cyclohexanon mit Hydroxylamin oder einem Salz des Hydroxylamins nach bekannten Methoden in das 4-(4-tert.-Butylphenyl)cyclohexanonoxim überführt und dieses Oxim anschließend mit Natrium in Ethanol (vgl, z.B. Chem. Ind. (London) 1972, 683) reduziert. Das dabei entstehende trans-4-(tert.-Butylphenyl)cyclohexylamin wird dann in einem weiteren Schritt mit einer Carbonylverbindung der Formel

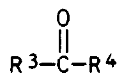 4

in der $R^3$ und $R^4$ so beschaffen sind, daß der Rest

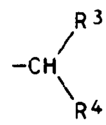

in seiner Gesamtheit dem Rest $R^2$ mit seinen oben genannten Bedeutungen entspricht, umgesetzt und das dabei gebildete Imin direkt oder nach Isolierung mit einem Reduktionsmittel zum Amin der Formel 1 reduziert.

Reduziert man das 4-(4-tert.-Butylphenyl)cyclohexanonoxim beispielsweise mit Wasserstoff, so erhält man ein Gemisch aus cis- und trans-4-(4-tert.-Butylphenyl)cyclohexylamin, welches nach an sich bekannten Verfahren, beispielsweise durch Chromatographie, Destillation oder durch fraktionierende Kristallisation eines Säureadditionssalzes mit anschließender Freisetzung der Base in die reinen cis- bzw. trans-Isomeren getrennt werden kann. Beide Isomere können nun für sich jeweils mit einer Carbonylverbindung der Formel

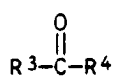 4

in der $R^3$ und $R^4$ die oben genannte Bedeutung haben, umgesetzt werden, um zu den reinen cis- bzw. trans-Konfigurierten Aminen der Formel 1 zu gelangen.

Selbstverständlich kann man auch das Gemisch aus cis- und trans-4-(4-tert.-Butylphenyl)cyclohexylamin mit einer Carbonylverbindung der Formel 4 umsetzen und ggf. das entstandene Isomerengemisch von Aminen der Formel 1 in seine Bestandteile trennen, z.B. durch Chromatographie oder Kristallisation.

Eine weitere Möglichkeit zur Herstellung eines cis-/trans-Gemisches von 4-(4-tert.-Butylphenyl)cyclohexylamin besteht darin, daß man cis-/trans-4-Phenylcyclohexylamin mit 2-Methylpropen in Gegenwart einer mindestens äquimolaren Menge Mineralsäure, beispielsweise Schwefelsäure, alkyliert. Als Lösungsmittel kommen bevorzugt chlorierte Kohlenwasserstoffe wie z.B. Dichlormethan, Tetrachlormethan, 1,2-Dichlorethan zum Einsatz. Die Alkylierung kann bei Raumtemperatur oder darunter, vorzugsweise bei 0-20°C durchgeführt werden.

Das als Ausgangsprodukt verwendete 4-Phenylcyclohexylamin ist bekannt. Es kann beispielsweise durch Hydrieren von 4-Aminobiphenyl (R. Egli, C.H. Eugster, Helv. Chim. Acta 58 (1975) 2321) oder von 4-Phenylcyclohexanonoxim (Nightingale et.al., J. Org. Chem. 17 (1952) 1017) hergestellt werden.

Die für das Verfahren b) benötigten Carbonylverbindungen der Formel 4 sind gängige Chemikalien und, falls nicht im Handel erhältlich, nach allgemein bekannten Methoden herstellbar.

Die beim Verfahren a) näher erläuterten Reaktionsbedingungen gelten sinngemäß auch für das Verfahren b).

Nach dem oben beschriebenen Verfahren b) erhält man die Amine der Formel 1, in denen $R^1$ für Wasserstoff steht. Diese sekundären Amine können gegebenenfalls durch bekannte Alkylierungsreaktionen in tertiäre Amine der Formel 1, in denen $R^1$ für $C_1$-$C_6$-Alkyl oder $C_3$-$C_6$-Alkenyl steht, überführt werden.

Verwendet man als Alkylierungsmittel eine Methylverbindung der Formel

$$CH_3\text{-}A \quad 5,$$

in der A für eine nucleofuge Austrittsgruppe, z.B. für Chlor, Brom, Jod oder eine der Gruppen $O\text{-}SO_2\text{-}OCH_3$, $O\text{-}SO_2\text{-}CH_3$, $O\text{-}SO_2\text{-}p\text{-}C_6H_4\text{-}CH_3$ steht, und setzt dieses Alkylierungsmittel im überschuß ein, so erhält man 4-(4-tert.-Butylphenyl)cyclohexylammoniumsalze der Formel 2 in denen $R^2$ die oben genannten Bedeutungen hat.

Diese Quarternisierung kann mit oder ohne Verdünnungsmittel durchgeführt werden. Als Verdünnungsmittel eignen sich beispielsweise Alkohole wie Methanol, Ethanol, n- oder iso-Propanol, n-Butanol, Cyclohexanol, Ether wie Diethylether, Methyl-tert.-butylether, Tetrahydrofuran, Dioxan, Ester wie Essigsäuremethylester, Essigsäureethylester, Nitrile wie Acetonitril, Propionitril, Nitroverbindungen wie Nitromethan, Nitrobenzol oder andere, bezüglich dieser Reaktion inerte Lösungsmittel. Die Alkylierung wird vorzugsweise mit zwei bis 6-facher molarer Menge an Alkylierungsmittel der Formel 5 und bevorzugt in einem Temperaturintervall von 20 bis 200°C durchgeführt. Zweckmäßig arbeitet man bei der Siedetemperatur des jeweiligen Verdünnungsmittels, oder, falls ohne ein solches gearbeitet wird, des jeweiligen Alkylierungsmittels.

Es ist zuweilen vorteilhaft, die Quaternisierung in Gegenwart einer Hilfsbase durchzuführen. Dabei kann die Base im überschuß oder, bezüglich des zu alkylierenden Amins 1, in äquimolarer Menge zugegeben werden. Besonders vorteilhaft ist die Verwendung einer im Reaktionsmedium unlöslichen Base wie beispielsweise Natrium-, Kalium-oder Calciumcarbonat.

Als Reaktionsprodukt erhält man ein 4-(4-tert.-Butylphenyl)-cyclohexylammoniumsalz der Formel

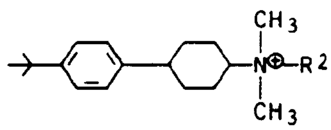

$A^{\ominus}$ 2a

in der $R^2$ und A die oben genannten Bedeutungen haben. Bei Bedarf kann das Anion $A^{\ominus}$ durch übliche Methoden, beispielsweise durch Ionenaustauschchromatographie durch ein anderes, pflanzenphysiologisch verträgliches Säureanionen ersetzt werden.

Verbindungen gibt Schema 1

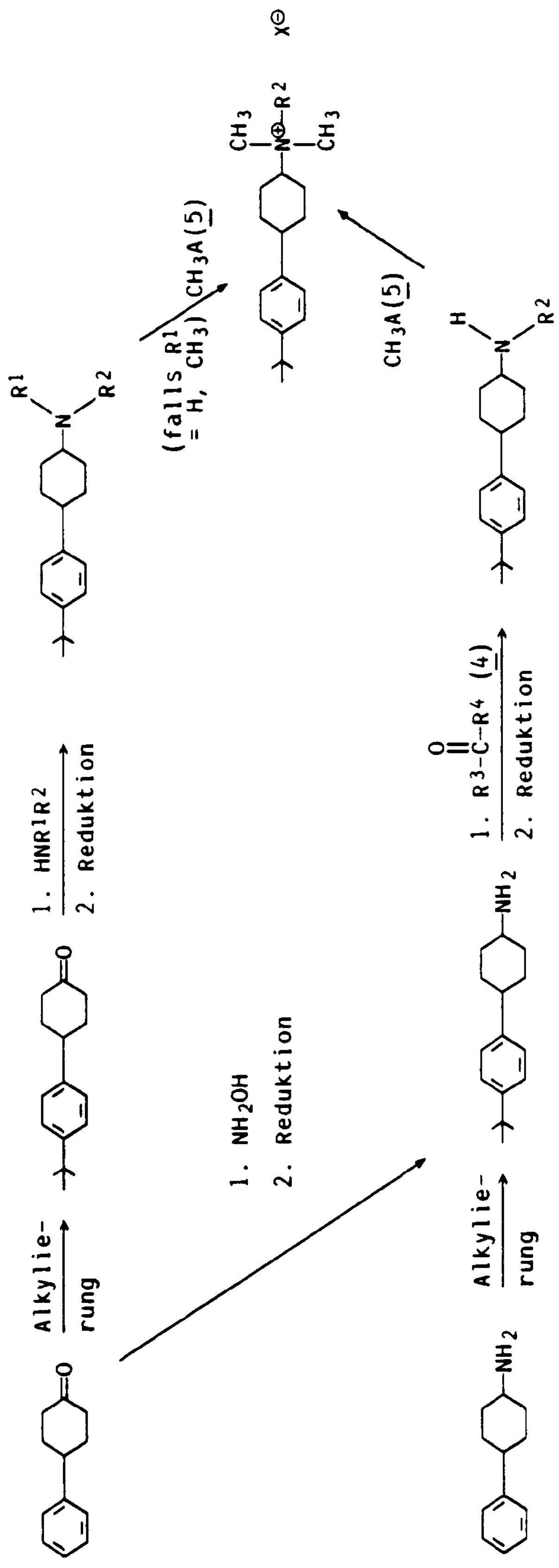


Die folgenden Beispiele sollen die Herstellung der erfindungsgemäßen Verbindungen näher erläutern:

Beispiel 1

4-(4-tert.-Butylphenyl)cyclohexanon

Zu einer Lösung von 50 g (0,287 mol) 4-Phenylcyclohexanon in 600 ml Dichlormethan tropft man unter Rühren und Kühlen bei 0-5°C 170,5 g (1,74 mol) konz. Schwefelsäure. In diese Mischung gast man innerhalb von 15 - 20 min bei 10 - 15°C 19,5 g (0,35 mol) Isobutylen (2-Methylpropen) ein. Man rührt eine Stunde bei Raumtemp. (20°C) nach und tropft dann unter Eiskühlung 250 ml Wasser zu. Die organische Phase wird abgetrennt, mit Wasser, 10 proz. NaOH und nochmals mit Wasser neutral gewaschen, über $Na_2SO_4$ getrocknet und i. Vak. eingeengt. Der Rückstand wird aus n-Pentan umkristallisiert. 50,7 g (76 % d. Th.) farblose Kristalle vom Schmp. 80 -82°C.

$^1$H-NMR ($CDCl_3$): $\delta$ = 7, 35(d, 2H), 7,18(d, 2H), 3, 00(br,t,1H), 2, 50(m,4H), 2,21(m,2H), 1,95(m,2H), 1,30(s,9H).
IR (KBr): $\nu$ = 2965,2944,2867,1710,1418, 1160,832,808,570 $cm^{-1}$.

Beispiel 2

4-trans-tert.-Butyl-4'-cis-/trans-(4-tert.-butylphenyl)-N,N-dicyclohexylamin (Verbindung Nr. 62)

16, 1 g (0,07 mol) 4-(4-tert.-Butylphenyl)cyclohexanon, 21,7 g (0,14 mol) trans-4-tert.-Butylcyclohexylamin und 4,8 g (0,035 mol) wasserfreies Zinkchlorid werden in 250 ml Methanol gelöst. In die Lösung werden 4,4 g (0,07 mol) Natriumcyanborhydrid portionsweise eingetragen. Man rührt 24 Stunden bei Raumtemp. und zieht dann die Hauptmenge des Lösungsmittels i. Vak. ab. Der Rückstand wird in Wasser aufgenommen, mit konz. NaOH alkalisch gestellt und mit Methyl-tert.-butylether mehrfach extrahiert. Die organische Phase wird nach Trocknen über $Na_2SO_4$ zunächst vom Lösungsmittel befreit, dann i. Vak, destilliert. Nach einem Vorlauf, der hauptsächlich aus 4-tert.-Butylcyclohexylamin besteht, erhält man bei 210 - 212°C/0,2 mbar 19,3 g (75 % d. Th.) der Titelverbindung als farbloses, zähes öl. Nach GC- und $^1$H-NMR-Analyse handelt es sich um ein cis-/trans-Gemisch im Verhältnis 2:3.

| | | | | | |
|---|---|---|---|---|---|
| $C_{26}H_{43}N$ (369,1) | Ber. | C 84,48 | H 11,73 | N 3,79 |
| | Gef. | C 84,5 | H 11,7 | N 3,6 |

Beispiel 3

Isomerentrennung von 4-trans-tert.-Butyl-4'-cis-/trans-(4-tert.-butylphenyl )-N, N-dicyclohexylamin

8,0 g eines gemäß Beispiel 2 erhaltenen cis-/trans-Isomerengemischs werden durch Chromatographie an einer 30 x 3 cm Kieselgelsäule (Macherey & Nagel, 0,04 - 0,06 mm) mit Cyclohexan/Essigsäureethylester (4:1) unter 0,1 bar Stickstoff getrennt. Man eluiert zunächst das 4-trans-tert.-Butyl-4'-cis-(4-tert.-butylphenyl)-N,N-dicyclo-hexylamin als farblosen Feststoff vom Schmp. 54 - 56°C (Ethanol). Die relative Stereochemie wurde durch $^1$H- und $^{13}$C-NMR-Spektroskopie bestimmt.

$^1$H-NMR ($CDCl_3$): u.a. $\delta$ = 3,00 (m,1'-$H_{eq}$), 2,55 (m,1-$H_{ax}$), 2,40 (4'-$H_{ax}$).

$^{13}$C-NMR ($CDCl_3$): u.a. $\delta$ = 54,04 (C-1'), 43,69 (C-4'), 34,86 (C-2 '(6')), 33,39 (C-3' (5')).

Nach einer Mischfraktion (0,8 g) erhält man das 4-trans-tert.-Butyl-4'-trans-(4-tert.-butylphenyl)-N,N-dicyclohexylamin zunächst als blaßgelbes öl, das aus Acetonitril kristallisiert. Farblose Kristalle, Schmp. 112-113°C.

$^1$H-NMR ($CDCl_3$): u.a. $\delta$ = 2,67 (br.m, 1'-$H_{ax}$), 2,50 (br.m, 1-$H_{ax}$ + 4'$H_{ax}$).

$^{13}$C-NMR ($CDCl_3$): u.a. $\delta$ = 48,42 (C-1'), 42,69 (C-4'), 30,98 (C-2'(6')), 28,30 (C-3'(5')).

Beispiel 4

N,N-Dimethyl-4-trans-tert.-butyl-4'-cis-/trans(4-tert.-butylphenyl )-N, N-dicyclohexylammoniumjodid

7,4 g (0,02 mol) 4-trans-tert.-Butyl-4'-cis-/trans-(4-tert.-butylphenyl)-N,N-dicyclohexylamin (Bsp. 2), 11,4 g (0,08 mol) Methyljodid und 10,2 g Natriumcarbonat (0,10 mol) werden in 50 ml Ethanol 6 Stunden unter Rückfluß erhitzt. Nach Erkalten wird filtriert, das Filtrat i. Vak. zur Trockne eingeengt und der ölige Rückstand mit 50 ml Essigsäureethylester aufgekocht. Man läßt über Nacht stehen, saugt den ausgefallenen NiederSchlag ab,

wäscht mit eiskaltem Aceton nach und trocknet i. Vak. bei 50°C. 6,7 g (62 % d. Th.) Quartärsalz als farblose Kristalle vom Schmp. 184-185°C.

Beispiel 5

cis-/trans/4-(4-tert.-Butylphenyl)cyclohexylamin

Zu 52,5 g (0,30 mol) 4-Phenylcyclohexylamin (cis-/trans-Verhältnis ca. 2:3) tropft man unter guter Kühlung bei 0-5°C 165 g konz. $H_2SO_4$ (1,68 mol). Man verdünnt mit 1 000 ml Dichlormethan und gast bei 10-15°C 21,8 g (0,39 mol) Isobutylen innerhalb von 20 Minuten ein. Nach einer Stunde bei 20°C werden unter Eiskühlung 250 ml Wasser zugetropft. Die wässrige Phase wird noch 2 x mit $CH_2Cl_2$ extrahiert. Die vereinigten organ. Phasen werden mit 1 1 10 proz. (Gew.-%)NaOH intensiv verrührt, dann mit Wasser gewaschen, über $Na_2SO_4$ getrocknet und i. Vak. eingedampft. Das laut Gaschromatogramm ca. 70 prozentige Rohamin wird in 500 ml Ether gelöst und durch Eingasen von HCl unter Eiskühlung als Hydrochlorid ausgefällt. 35,3 g (44,5 % d. Th.) gelbliches Kristallpulver, das sich oberhalb 240°C zersetzt.

IR (KBr): $\nu$ = 2956,2932,2868,1607,1510,1448,1362,1268,831, 574 $cm^{-1}$.

Die freie Base erhält man daraus durch Versetzen mit wässriger Ammoniaklösung, Extraktion mit Methyl-tert.-butylether, Trocknen und Einengen der organischen Phase als farbloses Öl (cis-trans-Gemisch). Charakteristische NMR-Daten ($CDCl_3$): cis-4-(4-tert.-Butylphenyl)cyclohexylamin: $\delta$ 1-$H_{eq}$ = 3,19; $\gamma$ C-1 = 45,3 ppm.

trans-4-(4-tert.-Butylphenyl)cyclohexylamin:

$\delta$ $^1$-$H_{ax}$ = 2,70; $\delta$ C-1 = 50,2 ppm.

Beispiel 6

N-Benzyl-N-4-(4-tert.-butylphenyl)cyclohexylamin (Verbindung Nr. 104)

Die Lösung von 11,6 g (0,05 mol) 4-(4-tert.-Butylphenyl)cyclohexylamin (cis-/trans-Gemisch) und 6,4 g (0,06 mol) frisch destilliertem Benzaldehyd in 200 ml Toluol wird mit 14,2 g (0,10 mol) Natriumsulfat versetzt und über Nacht bei Raumtemp. gerührt. Nach Filtration wird das Toluol abgezogen und durch Ethanol ersetzt. Man gibt nun 2,7 g (0,07 mol) Natriumborhydrid zu, kocht eine Stunde unter Rückfluß, engt zur Trockene ein und verteilt den Rückstand zwischen Wasser und Methyl-tert.-butylether. Die organische Phase wird mit Wasser gewaschen, über Natriumsulfat getrocknet, eingeengt und bei 2 mbar bis 200°C andestilliert. 9 g (56 % d. Th.) schwach rötliches Harz, nach $^1$H-NMR und Gaschromatogramm ein 2:1-trans-/cis-Isomerengemisch.

IR (Film): $\nu$ =2961,2927,2862,1461,1452,1362,827,736,698,572 $cm^{-1}$. $C_{23}H_{31}N$ (321,1)

Ber. C 85,92 H 9,72 H 4,36

Gef. C 85,5 H 9,9 H 4,1

Entsprechend diesen Beispielen können folgende Verbindungen hergestellt werden:

| Nr. | $R^1$ | $R^2$ | Physik. Daten |
|---|---|---|---|
| 1 | H | Methyl | |
| 2 | H | Ethyl | |
| 3 | H | 1-Propyl | |
| 4 | H | 2-Propyl | |
| 5 | H | 1-Butyl | Kp. 220-224°C/2 mbar (cis/trans=1:2) |
| 6 | H | 2-Butyl | |
| 7 | H | tert.-Butyl | |
| 8 | H | 2-Methyl-1-propyl | |
| 9 | H | 1-Pentyl | |
| 10 | H | 2-Methyl-1-butyl | |
| 11 | H | 2-Methyl-1-pentyl | |
| 12 | H | 2-Ethyl-1-butyl | |
| 13 | H | 2,2-Dimethyl-1-propyl | |
| 14 | H | 3,3-Dimethyl-1-butyl | |
| 15 | H | 3-Methyl-1-butyl | Harz (cis/trans=1:2) |
| 16 | H | 1-Hexyl | |
| 17 | H | 2-Methyl-1-hexyl | |
| 18 | H | 2-Ethyl-1-hexyl | |
| 19 | H | 2,4,4-Trimethyl-1-pentyl | |
| 20 | H | 1-Heptyl | |
| 21 | H | 1-Octyl | |
| 22 | H | 1-Nonyl | |
| 23 | H | 1-Decyl | |
| 24 | H | 1-Undecyl | |
| 25 | H | 1-Dodecyl | |
| 26 | H | 2-Propen-1-yl | |
| 27 | H | 2-Buten-1-yl | |

| Nr. | $R^1$ | $R^2$ | Physik. Daten |
|---|---|---|---|
| 28 | H | 2-Penten-1-yl | |
| 29 | H | 2-Hexen-1-yl | |
| 30 | H | 3-Methyl-2-buten-1-yl | |
| 31 | H | 2,3-Dimethyl-2-buten-1-yl | |
| 32 | H | 2-Hydroxyethyl | |
| 33 | H | 6-Hydroxy-1-hexyl | |
| 34 | H | 3-Chlor-1-butyl | |
| 35 | H | 4-Chlor-1-butyl | |
| 36 | H | 3-Chlor-2-methyl-1-propyl | |
| 37 | H | Cyclopropyl | |
| 38 | H | Cyclobutyl | |
| 39 | H | Cyclopentyl | Kp. 180-186°C/0,5 mbar (cis/trans=1:2) |
| 40 | H | Cyclohexyl | Kp. 167-170°C/0,4 mbar (cis/trans=1:2) |
| 41 | H | Cycloheptyl | |
| 42 | H | Cyclooctyl | |
| 43 | H | Cyclodecyl | |
| 44 | H | Cyclododecyl | |
| 45 | H | 1-Methylcyclopropyl | |
| 46 | H | 1-Methylcyclopentyl | |
| 47 | H | 1-Methylcyclohexyl | |
| 48 | H | 2-Methylcyclohexyl | |
| 49 | H | 3-Methylcyclohexyl | |
| 50 | H | 4-Methylcyclohexyl | IR: 2949,2923,2865,2851,1447,1363,1112,826,571 |
| 51 | H | 2,2-Dimethylcyclohexyl | |
| 52 | H | 3,3-Dimethylcyclohexyl | |
| 53 | H | 4,4-Dimethylcyclohexyl | |
| 54 | H | 2,6-Dimethylcyclohexyl | |

| Nr. | $R^1$ | $R^2$ | Physik. Daten |
|---|---|---|---|
| 55 | H | 3,3,5-Trimethylcyclohexyl | |
| 56 | H | 3,3,5,5-Tetramethylcyclohexyl | |
| 57 | H | 4-Ethylcyclohexyl | |
| 58 | H | 4-Propylcyclohexyl | |
| 59 | H | 4-Isopropylcyclohexyl | Hydrochlorid: IR:2951,2865,2794 2734,1462,1385,1363,827,572 |
| 60 | H | 4-tert.-Butylcyclohexyl | |
| 61 | H | cis-4-tert.-Butylcyclohexyl | |
| 62 | H | trans-4-tert.-Butylcyclohexyl | 4'-cis: Fp. 54-56°C; 4'-trans: Fp. 112-113°C |
| 63 | H | 4-tert.-Amylcyclohexyl | |
| 64 | H | Bicyclo[2.2.1]-hept-2-yl | |
| 65 | H | 1,7,7-Trimethyl-bicyclo[2.2.1]-hept-2-yl | |
| 66 | H | Bicyclo[4.4.0]dec-2-yl | |
| 67 | H | Bicyclo[4.4.0]dec-3-yl | IR: 2923,2856,1464,1447,1362,1269,1111,826,571 Harz (cis/trans=1:2) |
| 68 | H | 2,6,6-Trimethylbicyclo[3.1.1]-hept-3-yl | |
| 69 | H | Phenyl | IR:2960,2928,2863,1602,1503,1362,1312,827,746, 691 |
| 70 | H | 2-Methylphenyl | |
| 71 | | 3-Methylphenyl | |
| 72 | H | 4-Methylphenyl | |
| 73 | H | 2,4-Dimethylphenyl | |
| 74 | H | 4-Isopropylphenyl | |
| 75 | H | 4-tert.-Butylphenyl | IR: 2961,2928,2864,1615,1519,1460,1445,1363, 1269,1193,819 |

| Nr. | $R^1$ | $R^2$ | Physik. Daten |
|---|---|---|---|
| 76 | H | 2-Methoxyphenyl | IR: 2960, 2931, 2863, 1602, 1519, 1512, 1456, 1247, 1222, 734 |
| 77 | H | 4-Methoxyphenyl | IR: 2952, 2925, 2863, 1512, 1461, 1458, 1255, 1248, 1031, 823 |
| 78 | H | 3,4-Dimethoxyphenyl | |
| 79 | H | 4-tert.-Butoxyphenyl | |
| 80 | H | 2-Trifluormethylphenyl | |
| 81 | H | 3-Trifluormethylphenyl | |
| 82 | H | 4-Trifluormethylphenyl | |
| 83 | H | 4-Trifluormethoxyphenyl | |
| 84 | H | 2-Fluorphenyl | IR: 2961, 2930, 2864, 1620, 1521, 1512, 1449, 1249, 1185, 820, 740 |
| 85 | H | 3-Fluorphenyl | |
| 86 | H | 4-Fluorphenyl | IR: 2960, 2931, 2865, 1508, 1540, 1220, 1211, 823, 711, 571 |
| 87 | H | 2,4-Difluorphenyl | |
| 88 | H | 2-Chlorphenyl | |
| 89 | H | 2-Chlor-4-fluorphenyl | |
| 90 | H | 3-Chlorphenyl | |
| 91 | H | 4-Chlorphenyl | IR: 2960, 2929, 2863, 1600, 1498, 1448, 1362, 1314, 826, 814 |
| 92 | H | 2,4-Dichlorphenyl | IR: 2960, 2929, 2864, 1592, 1505, 1461, 1449, 1362, 1321, 827 |
| 93 | H | 2,6-Dichlorphenyl | |
| 94 | H | 3,5-Dichlorphenyl | |
| 95 | H | 2-Cyanphenyl | |
| 96 | H | 3-Cyanphenyl | |

| Nr. | $R^1$ | $R^2$ | Physik. Daten |
|---|---|---|---|
| 97 | H | 4-Cyanphenyl | |
| 98 | H | 2-Hydroxyphenyl | |
| 99 | H | 3-Hydroxyphenyl | |
| 100 | H | 4-Hydroxyphenyl | |
| 101 | H | 2-Nitrophenyl | |
| 102 | H | 3-Nitrophenyl | |
| 103 | H | 4-Nitrophenyl | |
| 104 | H | Benzyl | IR: 2961, 2927, 2862, 1461, 1452, 1362, 827, 736, 698, 572 |
| 105 | H | 2-Methylbenzyl | |
| 106 | H | 4-Methylbenzyl | |
| 107 | H | 2,4-Dimethylbenzyl | |
| 108 | H | 4-Isopropylbenzyl | |
| 109 | H | 4-tert.-Butylbenzyl | IR: 2961, 2925, 2863, 1509, 1460, 1448, 1362, 825, 571 |
| 110 | H | 2-Methoxybenzyl | |
| 111 | H | 3-Methoxybenzyl | |
| 112 | H | 4-Methoxybenzyl | |
| 113 | H | 3,4-Dimethoxybenzyl | |
| 114 | H | 4-tert.-Butoxybenzyl | |
| 115 | H | 2-Trifluormethylbenzyl | |
| 116 | H | 3-Trifluormethylbenzyl | |
| 117 | H | 4-Trifluormethylbenzyl | |
| 118 | H | 4-Trifluormethoxybenzyl | |
| 119 | H | 2-Fluorbenzyl | IR: 2961, 2927, 28 64, 1508, 1490, 1456, 1363, 1229, 828, 756 |
| 120 | H | 3-Fluorbenzyl | |

| Nr. | $R^1$ | $R^2$ | Physik. Daten |
|---|---|---|---|
| 121 | H | 4-Fluorbenzyl | IR: 2960,2927,2864,1509,1462,1449,1362, 1221,827,572 |
| 122 | H | 2,4-Difluorbenzyl | |
| 123 | H | 2-Chlor-4-fluorbenzyl | |
| 124 | H | 2-Chlorbenzyl | |
| 125 | H | 3-Chlorbenzyl | |
| 126 | H | 4-Chlorbenzyl | |
| 127 | H | 2,4-Dichlorbenzyl | Hydrochlorid: IR: 2955,2863,2766,2721, 2574,1478,1469,825 |
| 128 | H | 2,6-Dichlorbenzyl | |
| 129 | H | 3,5-Dichlorbenzyl | |
| 130 | H | 2-Cyanbenzyl | |
| 131 | H | 4-Cyanbenzyl | |
| 132 | H | 2-Hydroxybenzyl | |
| 133 | H | 3-Hydroxybenzyl | |
| 134 | H | 4-Hydroxybenzyl | |
| 135 | H | 2-Nitrobenzyl | |
| 136 | H | 3-Nitrobenzyl | |
| 137 | H | 4-Nitrobenzyl | |
| 138 | H | 2,4-Dinitrobenzyl | |
| 139 | H | 2-Phenylethyl | |
| 140 | H | 2-(4-Methylphenyl)ethyl | |
| 141 | H | 2-(4-tert.-Butylphenyl)ethyl | |
| 142 | H | 2-(2-Methoxyphenyl)ethyl | |
| 143 | H | 2-(4-Methoxyphenyl)ethyl | |
| 144 | H | 2-(3,4-Dimethoxyphenyl)ethyl | |
| 145 | H | 2-(4-tert.-Butoxyphenyl)ethyl | |

| Nr. | $R^1$ | $R^2$ | Physik. Daten |
|---|---|---|---|
| 146 | H | 2-(4-Trifluormethoxyphenyl)ethyl | |
| 147 | H | 2-(3-Trifluormethylphenyl)ethyl | |
| 148 | H | 2-(2-Fluorphenyl)ethyl | |
| 149 | H | 2-(4-Fluorphenyl)ethyl | |
| 150 | H | 2-(2-Chlorphenyl)ethyl | |
| 151 | H | 2-(4-Chlorphenyl)ethyl | |
| 152 | H | 2-(2,4-Dichlorphenyl)ethyl | |
| 153 | H | 2-(2-Chlor-4-fluorphenyl)ethyl | |
| 154 | H | 2-(4-Cyanophenyl)ethyl | |
| 155 | H | 2-(4-Hydroxyphenyl)ethyl | |
| 156 | H | 2-(4-Nitrophenyl)ethyl | |
| 157 | H | 3-Phenylpropyl | |
| 158 | H | 3-(4-Methylphenyl)propyl | |
| 159 | H | 3-(4-tert.-Butylphenyl)propyl | |
| 160 | H | 3-(2-Methoxyphenyl)propyl | |
| 161 | H | 3-(4-Methoxyphenyl)propyl | |
| 162 | H | 3-(3,4-Dimethoxyphenyl)propyl | |
| 163 | H | 3-(4-tert.-Butoxyphenyl)propyl | |
| 164 | H | 3-(3-Trifluormethylphenyl)propyl | |
| 165 | H | 3-(4-Trifluormethoxyphenyl)propyl | |
| 166 | H | 3-(2-Fluorphenyl)propyl | |
| 167 | H | 3-(4-Fluorphenyl)propyl | |
| 168 | H | 3-(2-Chlorphenyl)propyl | |
| 169 | H | 3-(4-Chlorphenyl)propyl | |
| 170 | H | 3-(2,4-Dichlorphenyl)propyl | |
| 171 | H | 3-(2-Chlor-4-fluorphenyl)propyl | |
| 172 | H | 3-(4-Cyanophenyl)propyl | |

| Nr. | $R^1$ | $R^2$ | Physik. Daten |
|---|---|---|---|
| 173 | H | 3-(4-Hydroxyphenyl)propyl | |
| 174 | H | 3-(4-Nitrophenyl)propyl | |
| 175 | Methyl | 1-Pentyl | |
| 176 | Methyl | 2-Methyl-1-butyl | |
| 177 | Methyl | 2-Methyl-1-pentyl | |
| 178 | Methyl | 2-Ethyl-1-butyl | |
| 179 | Methyl | 2,2-Dimethyl-1-propyl | |
| 180 | Methyl | 3,3-Dimethyl-1-butyl | Öl (cis/trans=1:3) |
| 181 | Methyl | 3-Methyl-1-butyl | |
| 182 | Methyl | 1-Hexyl | |
| 183 | Methyl | 2-Methyl-1-hexyl | |
| 184 | Methyl | 2-Ethyl-1-hexyl | |
| 185 | Methyl | 2,4,4-Trimethyl-1-pentyl | |
| 186 | Methyl | 1-Heptyl | |
| 187 | Methyl | 1-Octyl | |
| 188 | Methyl | 1-Nonyl | |
| 189 | Methyl | 1-Decyl | |
| 190 | Methyl | 1-Undecyl | |
| 191 | Methyl | 1-Dodecyl | |
| 192 | Methyl | 2-Propen-1-yl | |
| 193 | Methyl | 2-Buten-1-yl | |
| 194 | Methyl | 2-Penten-1-yl | |
| 195 | Methyl | 2-Hexen-1-yl | |
| 196 | Methyl | 3-Methyl-2-buten-1-yl | |
| 197 | Methyl | 2,3-Dimethyl-2-buten-1-yl | |
| 198 | Methyl | 2-Hydroxyethyl | |
| 199 | Methyl | 6-Hydroxy-1-hexyl | |

| Nr. | $R^1$ | $R^2$ | Physik. Daten |
|---|---|---|---|
| 200 | Methyl | 3-Chlor-1-butyl | |
| 201 | Methyl | 4-Chlor-1-butyl | |
| 202 | Methyl | 3-Chlor-2-methyl-1-propyl | |
| 203 | Methyl | Cyclopropyl | |
| 204 | Methyl | Cyclobutyl | |
| 205 | Methyl | Cyclopentyl | |
| 206 | Methyl | Cyclohexyl | |
| 207 | Methyl | Cycloheptyl | |
| 208 | Methyl | Cyclooctyl | |
| 209 | Methyl | Cyclodecyl | |
| 210 | Methyl | Cyclododecyl | |
| 211 | Methyl | 1-Methylcyclopropyl | |
| 212 | Methyl | 1-Methylcyclopentyl | |
| 213 | Methyl | 1-Methylcyclohexyl | |
| 214 | Methyl | 2-Methylcyclohexyl | |
| 215 | Methyl | 3-Methylcyclohexyl | |
| 216 | Methyl | 4-Methylcyclohexyl | |
| 217 | Methyl | 2,2-Dimethylcyclohexyl | |
| 218 | Methyl | 3,3-Dimethylcyclohexyl | |
| 219 | Methyl | 4,4-Dimethylcyclohexyl | |
| 220 | Methyl | 2,6-Dimethylcyclohexyl | |
| 221 | Methyl | 3,3,5-Trimethylcyclohexyl | |
| 222 | Methyl | 3,3,5,5-Tetramethylcyclohexyl | |
| 223 | Methyl | 4-Ethylcyclohexyl | |
| 224 | Methyl | 4-Propylcyclohexyl | |
| 225 | Methyl | 4-Isopropylcyclohexyl | |
| 226 | Methyl | 4-tert.-Butylcyclohexyl | |

| Nr. | $R^1$ | $R^2$ | Physik. Daten |
|---|---|---|---|
| 227 | Methyl | cis-4-tert.-Butylcyclohexyl | |
| 228 | Methyl | trans-4-tert.-Butylcyclohexyl | |
| 229 | Methyl | 4-tert.-Amylcyclohexyl | |
| 230 | Methyl | Bicyclo[2.2.1]-hept-2-yl | |
| 231 | Methyl | 1,7,7-Trimethyl-bicyclo[2.2.1]-hept-2-yl | |
| 232 | Methyl | Bicyclo[4.4.0]dec-2-yl | |
| 233 | Methyl | Bicyclo[4.4.0]dec-3-yl | |
| 234 | Methyl | 2,6,6-Trimethylbicyclo[3.1.1]-hept-3-yl | |
| 235 | Methyl | Phenyl | |
| 236 | Methyl | 2-Methylphenyl | |
| 237 | Methyl | 3-Methylphenyl | |
| 238 | Methyl | 4-Methylphenyl | |
| 239 | Methyl | 2,4-Dimethylphenyl | |
| 240 | Methyl | 4-Isopropylphenyl | |
| 241 | Methyl | 4-tert.-Butylphenyl | |
| 242 | Methyl | 2-Methoxyphenyl | |
| 243 | Methyl | 4-Methoxyphenyl | |
| 244 | Methyl | 3,4-Dimethoxyphenyl | |
| 245 | Methyl | 4-tert.-Butoxyphenyl | |
| 246 | Methyl | 2-Trifluormethylphenyl | |
| 247 | Methyl | 3-Trifluormethylphenyl | |
| 248 | Methyl | 4-Trifluormethylphenyl | |
| 249 | Methyl | 4-Trifluormethoxyphenyl | |
| 250 | Methyl | 2-Fluorphenyl | |
| 251 | Methyl | 3-Fluorphenyl | |
| 252 | Methyl | 4-Fluorphenyl | |
| 253 | Methyl | 2,4-Difluorphenyl | |

| Nr. | $R^1$ | $R^2$ | Physik. Daten |
|---|---|---|---|
| 254 | Methyl | 2-Chlorphenyl | |
| 255 | Methyl | 2-Chlor-4-fluorphenyl | |
| 256 | Methyl | 3-Chlorphenyl | |
| 257 | Methyl | 4-Chlorphenyl | |
| 258 | Methyl | 2,4-Dichlorphenyl | |
| 259 | Methyl | 2,6-Dichlorphenyl | |
| 260 | Methyl | 3,5-Dichlorphenyl | |
| 261 | Methyl | 2-Cyanphenyl | |
| 262 | Methyl | 3-Cyanphenyl | |
| 263 | Methyl | 4-Cyanphenyl | |
| 264 | Methyl | 2-Hydroxyphenyl | |
| 265 | Methyl | 3-Hydroxyphenyl | |
| 266 | Methyl | 4-Hydroxyphenyl | |
| 267 | Methyl | 2-Nitrophenyl | |
| 268 | Methyl | 3-Nitrophenyl | |
| 269 | Methyl | 4-Nitrophenyl | |
| 270 | Methyl | Benzyl | |
| 271 | Methyl | 2-Methylbenzyl | |
| 272 | Methyl | 4-Methylbenzyl | |
| 273 | Methyl | 2,4-Dimethylbenzyl | |
| 274 | Methyl | 4-Isopropylbenzyl | |
| 275 | Methyl | 4-tert.-Butylbenzyl | |
| 276 | Methyl | 2-Methoxybenzyl | |
| 277 | Methyl | 3-Methoxybenzyl | |
| 278 | Methyl | 4-Methoxybenzyl | |
| 279 | Methyl | 3,4-Dimethoxybenzyl | |
| 280 | Methyl | 4-tert.-Butoxybenzyl | |

| Nr. | $R^1$ | $R^2$ | Physik. Daten |
|---|---|---|---|
| 281 | Methyl | 2-Trifluormethylbenzyl | |
| 282 | Methyl | 3-Trifluormethylbenzyl | |
| 283 | Methyl | 4-Trifluormethylbenzyl | |
| 284 | Methyl | 4-Trifluormethoxybenzyl | |
| 285 | Methyl | 2-Fluorbenzyl | |
| 286 | Methyl | 3-Fluorbenzyl | |
| 287 | Methyl | 4-Fluorbenzyl | |
| 288 | Methyl | 2,4-Difluorbenzyl | |
| 289 | Methyl | 2-Chlor-4-fluorbenzyl | |
| 290 | Methyl | 2-Chlorbenzyl | |
| 291 | Methyl | 3-Chlorbenzyl | |
| 292 | Methyl | 4-Chlorbenzyl | |
| 293 | Methyl | 2,4-Dichlorbenzyl | |
| 294 | Methyl | 2,6-Dichlorbenzyl | |
| 295 | Methyl | 3,5-Dichlorbenzyl | |
| 296 | Methyl | 2-Cyanbenzyl | |
| 297 | Methyl | 4-Cyanbenzyl | |
| 298 | Methyl | 2-Hydroxybenzyl | |
| 299 | Methyl | 3-Hydroxybenzyl | |
| 300 | Methyl | 4-Hydroxybenzyl | |
| 301 | Methyl | 2-Nitrobenzyl | |
| 302 | Methyl | 3-Nitrobenzyl | |
| 303 | Methyl | 4-Nitrobenzyl | |
| 304 | Methyl | 2,4-Dinitrobenzyl | |
| 305 | Methyl | 2-Phenylethyl | |
| 306 | Methyl | 2-(4-Methylphenyl)ethyl | |
| 307 | Methyl | 2-(4-tert.-Butylphenyl)ethyl | |

| Nr. | $R^1$ | $R^2$ | Physik. Daten |
|---|---|---|---|
| 308 | Methyl | 2-(2-Methoxyphenyl)ethyl | |
| 309 | Methyl | 2-(4-Methoxyphenyl)ethyl | |
| 310 | Methyl | 2-(3,4-Dimethoxyphenyl)ethyl | |
| 311 | Methyl | 2-(4-tert.-Butoxyphenyl)ethyl | |
| 312 | Methyl | 2-(4-Trifluormethoxyphenyl)ethyl | |
| 313 | Methyl | 2-(3-Trifluormethylphenyl)ethyl | |
| 314 | Methyl | 2-(2-Fluorphenyl)ethyl | |
| 315 | Methyl | 2-(4-Fluorphenyl)ethyl | |
| 316 | Methyl | 2-(2-Chlorphenyl)ethyl | |
| 317 | Methyl | 2-(4-Chlorphenyl)ethyl | |
| 318 | Methyl | 2-(2,4-Dichlorphenyl)ethyl | |
| 319 | Methyl | 2-(2-Chlor-4-fluorphenyl)ethyl | |
| 320 | Methyl | 2-(4-Cyanophenyl)ethyl | |
| 321 | Methyl | 2-(4-Hydroxyphenyl)ethyl | |
| 322 | Methyl | 2-(4-Nitrophenyl)ethyl | |
| 323 | Methyl | 3-Phenylpropyl | |
| 324 | Methyl | 3-(4-Methylphenyl)propyl | |
| 325 | Methyl | 3-(4-tert.-Butylphenyl)propyl | |
| 326 | Methyl | 3-(2-Methoxyphenyl)propyl | |
| 327 | Methyl | 3-(4-Methoxyphenyl)propyl | |
| 328 | Methyl | 3-(3,4-Dimethoxyphenyl)propyl | |
| 329 | Methyl | 3-(4-tert.-Butoxyphenyl)propyl | |
| 330 | Methyl | 3-(3-Trifluormethylphenyl)propyl | |
| 331 | Methyl | 3-(4-Trifluormethoxyphenyl)propyl | |
| 332 | Methyl | 3-(2-Fluorphenyl)propyl | |
| 333 | Methyl | 3-(4-Fluorphenyl)propyl | |
| 334 | Methyl | 3-(2-Chlorphenyl)propyl | |

| Nr. | $R^1$ | $R^2$ | Physik. Daten |
|---|---|---|---|
| 335 | Methyl | 3-(4-Chlorphenyl)propyl | |
| 336 | Methyl | 3-(2,4-Dichlorphenyl)propyl | |
| 337 | Methyl | 3-(2-Chlor-4-fluorphenyl)propyl | |
| 338 | Methyl | 3-(4-Cyanophenyl)propyl | |
| 339 | Methyl | 3-(4-Hydroxyphenyl)propyl | |
| 340 | Methyl | 3-(4-Nitrophenyl)propyl | |
| 341 | Ethyl | 1-Pentyl | |
| 342 | Ethyl | 2-Methyl-1-butyl | |
| 343 | Ethyl | 2-Methyl-1-pentyl | |
| 344 | Ethyl | 2-Ethyl-1-butyl | |
| 345 | Ethyl | 2,2-Dimethyl-1-propyl | |
| 346 | Ethyl | 3,3-Dimethyl-1-butyl | |
| 347 | Ethyl | 3-Methyl-1-butyl | |
| 348 | Ethyl | 1-Hexyl | |
| 349 | Ethyl | 2-Methyl-1-hexyl | |
| 350 | Ethyl | 2-Ethyl-1-hexyl | |
| 351 | Ethyl | 2,4,4-Trimethyl-1-pentyl | |
| 352 | Ethyl | 1-Heptyl | |
| 353 | Ethyl | 1-Octyl | |
| 354 | Ethyl | 1-Nonyl | |
| 355 | Ethyl | 1-Decyl | |
| 356 | Ethyl | 1-Undecyl | |
| 357 | Ethyl | 1-Dodecyl | |
| 358 | Ethyl | 2-Propen-1-yl | |
| 359 | Ethyl | 2-Buten-1-yl | |
| 360 | Ethyl | 2-Penten-1-yl | |
| 361 | Ethyl | 2-Hexen-1-yl | |

| Nr. | $R^1$ | $R^2$ | Physik. Daten |
|---|---|---|---|
| 362 | Ethyl | 3-Methyl-2-buten-1-yl | |
| 363 | Ethyl | 2,3-Dimethyl-2-buten-1-yl | |
| 364 | Ethyl | 2-Hydroxyethyl | |
| 365 | Ethyl | 6-Hydroxy-1-hexyl | |
| 366 | Ethyl | 3-Chlor-1-butyl | |
| 367 | Ethyl | 4-Chlor-1-butyl | |
| 368 | Ethyl | 3-Chlor-2-methyl-1-propyl | |
| 369 | Ethyl | Cyclopropyl | |
| 370 | Ethyl | Cyclobutyl | |
| 371 | Ethyl | Cyclopentyl | |
| 372 | Ethyl | Cyclohexyl | |
| 373 | Ethyl | Cycloheptyl | |
| 374 | Ethyl | Cyclooctyl | |
| 375 | Ethyl | Cyclodecyl | |
| 376 | Ethyl | Cyclododecyl | |
| 377 | Ethyl | 1-Methylcyclopropyl | |
| 378 | Ethyl | 1-Methylcyclopentyl | |
| 379 | Ethyl | 1-Methylcyclohexyl | |
| 380 | Ethyl | 2-Methylcyclohexyl | |
| 381 | Ethyl | 3-Methylcyclohexyl | |
| 382 | Ethyl | 4-Methylcyclohexyl | |
| 383 | Ethyl | 2,2-Dimethylcyclohexyl | |
| 384 | Ethyl | 3,3-Dimethylcyclohexyl | |
| 385 | Ethyl | 4,4-Dimethylcyclohexyl | |
| 386 | Ethyl | 2,6-Dimethylcyclohexyl | |
| 387 | Ethyl | 3,3,5-Trimethylcyclohexyl | |
| 388 | Ethyl | 3,3,5,5-Tetramethylcyclohexyl | |

| Nr. | $R^1$ | $R^2$ | Physik. Daten |
|---|---|---|---|
| 389 | Ethyl | 4-Ethylcyclohexyl | |
| 390 | Ethyl | 4-Propylcyclohexyl | |
| 391 | Ethyl | 4-Isopropylcyclohexyl | |
| 392 | Ethyl | 4-tert.-Butylcyclohexyl | |
| 393 | Ethyl | cis-4-tert.-Butylcyclohexyl | |
| 394 | Ethyl | trans-4-tert.-Butylcyclohexyl | Hydrochlorid Fp. 185-186°C |
| 395 | Ethyl | 4-tert.-Amylcyclohexyl | |
| 396 | Ethyl | Bicyclo[2.2.1]-hept-2-yl | |
| 397 | Ethyl | 1,7,7-Trimethyl-bicyclo[2.2.1]-hept-2-yl | |
| 398 | Ethyl | Bicyclo[4.4.0]dec-2-yl | |
| 399 | Ethyl | Bicyclo[4.4.0]dec-3-yl | |
| 400 | Ethyl | 2,6,6-Trimethylbicyclo[3.1.1]-hept-3-yl | |
| 401 | Ethyl | Phenyl | |
| 402 | Ethyl | 2-Methylphenyl | |
| 403 | Ethyl | 3-Methylphenyl | |
| 404 | Ethyl | 4-Methylphenyl | |
| 405 | Ethyl | 2,4-Dimethylphenyl | |
| 406 | Ethyl | 4-Isopropylphenyl | |
| 407 | Ethyl | 4-tert.-Butylphenyl | |
| 408 | Ethyl | 2-Methoxyphenyl | |
| 409 | Ethyl | 4-Methoxyphenyl | |
| 410 | Ethyl | 3,4-Dimethoxyphenyl | |
| 411 | Ethyl | 4-tert.-Butoxyphenyl | |
| 412 | Ethyl | 2-Trifluormethylphenyl | |
| 413 | Ethyl | 3-Trifluormethylphenyl | |
| 414 | Ethyl | 4-Trifluormethylphenyl | |
| 415 | Ethyl | 4-Trifluormethoxyphenyl | |

| Nr. | $R^1$ | $R^2$ | Physik. Daten |
|---|---|---|---|
| 416 | Ethyl | 2-Fluorphenyl | |
| 417 | Ethyl | 3-Fluorphenyl | |
| 418 | Ethyl | 4-Fluorphenyl | |
| 419 | Ethyl | 2,4-Difluorphenyl | |
| 420 | Ethyl | 2-Chlorphenyl | |
| 421 | Ethyl | 2-Chlor-4-fluorphenyl | |
| 422 | Ethyl | 3-Chlorphenyl | |
| 423 | Ethyl | 4-Chlorphenyl | |
| 424 | Ethyl | 2,4-Dichlorphenyl | |
| 425 | Ethyl | 2,6-Dichlorphenyl | |
| 426 | Ethyl | 3,5-Dichlorphenyl | |
| 427 | Ethyl | 2-Cyanphenyl | |
| 428 | Ethyl | 3-Cyanphenyl | |
| 429 | Ethyl | 4-Cyanphenyl | |
| 430 | Ethyl | 2-Hydroxyphenyl | |
| 431 | Ethyl | 3-Hydroxyphenyl | |
| 432 | Ethyl | 4-Hydroxyphenyl | |
| 433 | Ethyl | 2-Nitrophenyl | |
| 434 | Ethyl | 3-Nitrophenyl | |
| 435 | Ethyl | 4-Nitrophenyl | |
| 436 | Ethyl | Benzyl | |
| 437 | Ethyl | 2-Methylbenzyl | |
| 438 | Ethyl | 4-Methylbenzyl | |
| 439 | Ethyl | 2,4-Dimethylbenzyl | |
| 440 | Ethyl | 4-Isopropylbenzyl | |
| 441 | Ethyl | 4-tert.-Butylbenzyl | |
| 442 | Ethyl | 2-Methoxybenzyl | |

| Nr. | $R^1$ | $R^2$ | Physik. Daten |
|---|---|---|---|
| 443 | Ethyl | 3-Methoxybenzyl | |
| 444 | Ethyl | 4-Methoxybenzyl | |
| 445 | Ethyl | 3,4-Dimethoxybenzyl | |
| 446 | Ethyl | 4-tert.-Butoxybenzyl | |
| 447 | Ethyl | 2-Trifluormethylbenzyl | |
| 448 | Ethyl | 3-Trifluormethylbenzyl | |
| 449 | Ethyl | 4-Trifluormethylbenzyl | |
| 450 | Ethyl | 4-Trifluormethoxybenzyl | |
| 451 | Ethyl | 2-Fluorbenzyl | |
| 452 | Ethyl | 3-Fluorbenzyl | |
| 453 | Ethyl | 4-Fluorbenzyl | |
| 454 | Ethyl | 2,4-Difluorbenzyl | |
| 455 | Ethyl | 2-Chlor-4-fluorbenzyl | |
| 456 | Ethyl | 2-Chlorbenzyl | |
| 457 | Ethyl | 3-Chlorbenzyl | |
| 458 | Ethyl | 4-Chlorbenzyl | |
| 459 | Ethyl | 2,4-Dichlorbenzyl | |
| 460 | Ethyl | 2,6-Dichlorbenzyl | |
| 461 | Ethyl | 3,5-Dichlorbenzyl | |
| 462 | Ethyl | 2-Cyanbenzyl | |
| 463 | Ethyl | 4-Cyanbenzyl | |
| 464 | Ethyl | 2-Hydroxybenzyl | |
| 465 | Ethyl | 3-Hydroxybenzyl | |
| 466 | Ethyl | 4-Hydroxybenzyl | |
| 467 | Ethyl | 2-Nitrobenzyl | |
| 468 | Ethyl | 3-Nitrobenzyl | |
| 469 | Ethyl | 4-Nitrobenzyl | |

| Nr. | $R^1$ | $R^2$ | Physik. Daten |
|---|---|---|---|
| 470 | Ethyl | 2,4-Dinitrobenzyl | |
| 471 | Ethyl | 2-Phenylethyl | |
| 472 | Ethyl | 2-(4-Methylphenyl)ethyl | |
| 473 | Ethyl | 2-(4-tert.-Butylphenyl)ethyl | |
| 474 | Ethyl | 2-(2-Methoxyphenyl)ethyl | |
| 475 | Ethyl | 2-(4-Methoxyphenyl)ethyl | |
| 476 | Ethyl | 2-(3,4-Dimethoxyphenyl)ethyl | |
| 477 | Ethyl | 2-(4-tert.-Butoxyphenyl)ethyl | |
| 478 | Ethyl | 2-(4-Trifluormethoxyphenyl)ethyl | |
| 479 | Ethyl | 2-(3-Trifluormethylphenyl)ethyl | |
| 480 | Ethyl | 2-(2-Fluorphenyl)ethyl | |
| 481 | Ethyl | 2-(4-Fluorphenyl)ethyl | |
| 482 | Ethyl | 2-(2-Chlorphenyl)ethyl | |
| 483 | Ethyl | 2-(4-Chlorphenyl)ethyl | |
| 484 | Ethyl | 2-(2,4-Dichlorphenyl)ethyl | |
| 485 | Ethyl | 2-(2-Chlor-4-fluorphenyl)ethyl | |
| 486 | Ethyl | 2-(4-Cyanophenyl)ethyl | |
| 487 | Ethyl | 2-(4-Hydroxyphenyl)ethyl | |
| 488 | Ethyl | 2-(4-Nitrophenyl)ethyl | |
| 489 | Ethyl | 3-Phenylpropyl | |
| 490 | Ethyl | 3-(4-Methylphenyl)propyl | |
| 491 | Ethyl | 3-(4-tert.-Butylphenyl)propyl | |
| 492 | Ethyl | 3-(2-Methoxyphenyl)propyl | |
| 493 | Ethyl | 3-(4-Methoxyphenyl)propyl | |
| 494 | Ethyl | 3-(3,4-Dimethoxyphenyl)propyl | |
| 495 | Ethyl | 3-(4-tert.-Butoxyphenyl)propyl | |
| 496 | Ethyl | 3-(3-Trifluormethylphenyl)propyl | |

| Nr. | $R^1$ | $R^2$ | Physik. Daten |
|---|---|---|---|
| 497 | Ethyl | 3-(4-Trifluormethoxyphenyl)propyl | |
| 498 | Ethyl | 3-(2-Fluorphenyl)propyl | |
| 499 | Ethyl | 3-(4-Fluorphenyl)propyl | |
| 500 | Ethyl | 3-(2-Chlorphenyl)propyl | |
| 501 | Ethyl | 3-(4-Chlorphenyl)propyl | |
| 502 | Ethyl | 3-(2,4-Dichlorphenyl)propyl | |
| 503 | Ethyl | 3-(2-Chlor-4-fluorphenyl)propyl | |
| 504 | Ethyl | 3-(4-Cyanophenyl)propyl | |
| 505 | Ethyl | 3-(4-Hydroxyphenyl)propyl | |
| 506 | Ethyl | 3-(4-Nitrophenyl)propyl | |
| 507 | 1-Propyl | 1-Pentyl | |
| 508 | 1-Propyl | 2-Methyl-1-butyl | |
| 509 | 1-Propyl | 2-Methyl-1-pentyl | |
| 510 | 1-Propyl | 2-Ethyl-1-butyl | |
| 511 | 1-Propyl | 2,2-Dimethyl-1-propyl | |
| 512 | 1-Propyl | 3,3-Dimethyl-1-butyl | |
| 513 | 1-Propyl | 3-Methyl-1-butyl | |
| 514 | 1-Propyl | 1-Hexyl | |
| 515 | 1-Propyl | 2-Methyl-1-hexyl | |
| 516 | 1-Propyl | 2-Ethyl-1-hexyl | |
| 517 | 1-Propyl | 2,4,4-Trimethyl-1-pentyl | |
| 518 | 1-Propyl | 1-Heptyl | |
| 519 | 1-Propyl | 1-Octyl | |
| 520 | 1-Propyl | 1-Nonyl | |
| 521 | 1-Propyl | 1-Decyl | |
| 522 | 1-Propyl | 1-Undecyl | |
| 523 | 1-Propyl | 1-Dodecyl | |

| Nr. | $R^1$ | $R^2$ | Physik. Daten |
|---|---|---|---|
| 524 | 1-Propyl | 2-Propen-1-yl | |
| 525 | 1-Propyl | 2-Buten-1-yl | |
| 526 | 1-Propyl | 2-Penten-1-yl | |
| 527 | 1-Propyl | 2-Hexen-1-yl | |
| 528 | 1-Propyl | 3-Methyl-2-buten-1-yl | |
| 529 | 1-Propyl | 2,3-Dimethyl-2-buten-1-yl | |
| 530 | 1-Propyl | 2-Hydroxyethyl | |
| 531 | 1-Propyl | 6-Hydroxy-1-hexyl | |
| 532 | 1-Propyl | 3-Chlor-1-butyl | |
| 533 | 1-Propyl | 4-Chlor-1-butyl | |
| 534 | 1-Propyl | 3-Chlor-2-methyl-1-propyl | |
| 535 | 1-Propyl | Cyclopropyl | |
| 536 | 1-Propyl | Cyclobutyl | |
| 537 | 1-Propyl | Cyclopentyl | |
| 538 | 1-Propyl | Cyclohexyl | |
| 539 | 1-Propyl | Cycloheptyl | |
| 540 | 1-Propyl | Cyclooctyl | |
| 541 | 1-Propyl | Cyclodecyl | |
| 542 | 1-Propyl | Cyclododecyl | |
| 543 | 1-Propyl | 1-Methylcyclopropyl | |
| 544 | 1-Propyl | 1-Methylcyclopentyl | |
| 545 | 1-Propyl | 1-Methylcyclohexyl | |
| 546 | 1-Propyl | 2-Methylcyclohexyl | |
| 547 | 1-Propyl | 3-Methylcyclohexyl | |
| 548 | 1-Propyl | 4-Methylcyclohexyl | |
| 549 | 1-Propyl | 2,2-Dimethylcyclohexyl | |
| 550 | 1-Propyl | 3,3-Dimethylcyclohexyl | |

| Nr. | $R^1$ | $R^2$ | Physik. Daten |
|---|---|---|---|
| 551 | 1-Propyl | 4,4-Dimethylcyclohexyl | |
| 552 | 1-Propyl | 2,6-Dimethylcyclohexyl | |
| 553 | 1-Propyl | 3,3,5-Trimethylcyclohexyl | |
| 554 | 1-Propyl | 3,3,5,5-Tetramethylcyclohexyl | |
| 555 | 1-Propyl | 4-Ethylcyclohexyl | |
| 556 | 1-Propyl | 4-Propylcyclohexyl | |
| 557 | 1-Propyl | 4-Isopropylcyclohexyl | |
| 558 | 1-Propyl | 4-tert.-Butylcyclohexyl | |
| 559 | 1-Propyl | cis-4-tert.-Butylcyclohexyl | |
| 560 | 1-Propyl | trans-4-tert.-Butylcyclohexyl | Kp 230-236°C/0,2 mbar |
| 561 | 1-Propyl | 4-tert.-Amylcyclohexyl | |
| 562 | 1-Propyl | Bicyclo[2.2.1]-hept-2-yl | |
| 563 | 1-Propyl | 1,7,7-Trimethyl-bicyclo[2.2.1]-hept-2-yl | |
| 564 | 1-Propyl | Bicyclo[4.4.0]dec-2-yl | |
| 565 | 1-Propyl | Bicyclo[4.4.0]dec-3-yl | |
| 566 | 1-Propyl | 2,6,6-Trimethylbicyclo[3.1.1]-hept-3-yl | |
| 567 | 1-Propyl | Phenyl | |
| 568 | 1-Propyl | 2-Methylphenyl | |
| 569 | 1-Propyl | 3-Methylphenyl | |
| 570 | 1-Propyl | 4-Methylphenyl | |
| 571 | 1-Propyl | 2,4-Dimethylphenyl | |
| 572 | 1-Propyl | 4-Isopropylphenyl | |
| 573 | 1-Propyl | 4-tert.-Butylphenyl | |
| 574 | 1-Propyl | 2-Methoxyphenyl | |
| 575 | 1-Propyl | 4-Methoxyphenyl | |
| 576 | 1-Propyl | 3,4-Dimethoxyphenyl | |
| 577 | 1-Propyl | 4-tert.-Butoxyphenyl | |

| Nr. | $R^1$ | $R^2$ | Physik. Daten |
|---|---|---|---|
| 578 | 1-Propyl | 2-Trifluormethylphenyl | |
| 579 | 1-Propyl | 3-Trifluormethylphenyl | |
| 580 | 1-Propyl | 4-Trifluormethylphenyl | |
| 581 | 1-Propyl | 4-Trifluormethoxyphenyl | |
| 582 | 1-Propyl | 2-Fluorphenyl | |
| 583 | 1-Propyl | 3-Fluorphenyl | |
| 584 | 1-Propyl | 4-Fluorphenyl | |
| 585 | 1-Propyl | 2,4-Difluorphenyl | |
| 586 | 1-Propyl | 2-Chlorphenyl | |
| 587 | 1-Propyl | 2-Chlor-4-fluorphenyl | |
| 588 | 1-Propyl | 3-Chlorphenyl | |
| 589 | 1-Propyl | 4-Chlorphenyl | |
| 590 | 1-Propyl | 2,4-Dichlorphenyl | |
| 591 | 1-Propyl | 2,6-Dichlorphenyl | |
| 592 | 1-Propyl | 3,5-Dichlorphenyl | |
| 593 | 1-Propyl | 2-Cyanphenyl | |
| 594 | 1-Propyl | 3-Cyanphenyl | |
| 595 | 1-Propyl | 4-Cyanphenyl | |
| 596 | 1-Propyl | 2-Hydroxyphenyl | |
| 597 | 1-Propyl | 3-Hydroxyphenyl | |
| 598 | 1-Propyl | 4-Hydroxyphenyl | |
| 599 | 1-Propyl | 2-Nitrophenyl | |
| 600 | 1-Propyl | 3-Nitrophenyl | |
| 601 | 1-Propyl | 4-Nitrophenyl | |
| 602 | 1-Propyl | Benzyl | |
| 603 | 1-Propyl | 2-Methylbenzyl | |
| 604 | 1-Propyl | 4-Methylbenzyl | |

| Nr. | $R^1$ | $R^2$ | Physik. Daten |
|---|---|---|---|
| 605 | 1-Propyl | 2,4-Dimethylbenzyl | |
| 606 | 1-Propyl | 4-Isopropylbenzyl | |
| 607 | 1-Propyl | 4-tert.-Butylbenzyl | |
| 608 | 1-Propyl | 2-Methoxybenzyl | |
| 609 | 1-Propyl | 3-Methoxybenzyl | |
| 610 | 1-Propyl | 4-Methoxybenzyl | |
| 611 | 1-Propyl | 3,4-Dimethoxybenzyl | |
| 612 | 1-Propyl | 4-tert.-Butoxybenzyl | |
| 613 | 1-Propyl | 2-Trifluormethylbenzyl | |
| 614 | 1-Propyl | 3-Trifluormethylbenzyl | |
| 615 | 1-Propyl | 4-Trifluormethylbenzyl | |
| 616 | 1-Propyl | 4-Trifluormethoxybenzyl | |
| 617 | 1-Propyl | 2-Fluorbenzyl | |
| 618 | 1-Propyl | 3-Fluorbenzyl | |
| 619 | 1-Propyl | 4-Fluorbenzyl | |
| 620 | 1-Propyl | 2,4-Difluorbenzyl | |
| 621 | 1-Propyl | 2-Chlor-4-fluorbenzyl | |
| 622 | 1-Propyl | 2-Chlorbenzyl | |
| 623 | 1-Propyl | 3-Chlorbenzyl | |
| 624 | 1-Propyl | 4-Chlorbenzyl | |
| 625 | 1-Propyl | 2,4-Dichlorbenzyl | |
| 626 | 1-Propyl | 2,6-Dichlorbenzyl | |
| 627 | 1-Propyl | 3,5-Dichlorbenzyl | |
| 628 | 1-Propyl | 2-Cyanbenzyl | |
| 629 | 1-Propyl | 4-Cyanbenzyl | |
| 630 | 1-Propyl | 2-Hydroxybenzyl | |
| 631 | 1-Propyl | 3-Hydroxybenzyl | |

| Nr. | $R^1$ | $R^2$ | Physik. Daten |
|---|---|---|---|
| 632 | 1-Propyl | 4-Hydroxybenzyl | |
| 633 | 1-Propyl | 2-Nitrobenzyl | |
| 634 | 1-Propyl | 3-Nitrobenzyl | |
| 635 | 1-Propyl | 4-Nitrobenzyl | |
| 636 | 1-Propyl | 2,4-Dinitrobenzyl | |
| 637 | 1-Propyl | 2-Phenylethyl | |
| 638 | 1-Propyl | 2-(4-Methylphenyl)ethyl | |
| 639 | 1-Propyl | 2-(4-tert.-Butylphenyl)ethyl | |
| 640 | 1-Propyl | 2-(2-Methoxyphenyl)ethyl | |
| 641 | 1-Propyl | 2-(4-Methoxyphenyl)ethyl | |
| 642 | 1-Propyl | 2-(3,4-Dimethoxyphenyl)ethyl | |
| 643 | 1-Propyl | 2-(4-tert.-Butoxyphenyl)ethyl | |
| 644 | 1-Propyl | 2-(4-Trifluormethoxyphenyl)ethyl | |
| 645 | 1-Propyl | 2-(3-Trifluormethylphenyl)ethyl | |
| 646 | 1-Propyl | 2-(2-Fluorphenyl)ethyl | |
| 647 | 1-Propyl | 2-(4-Fluorphenyl)ethyl | |
| 648 | 1-Propyl | 2-(2-Chlorphenyl)ethyl | |
| 649 | 1-Propyl | 2-(4-Chlorphenyl)ethyl | |
| 650 | 1-Propyl | 2-(2,4-Dichlorphenyl)ethyl | |
| 651 | 1-Propyl | 2-(2-Chlor-4-fluorphenyl)ethyl | |
| 652 | 1-Propyl | 2-(4-Cyanophenyl)ethyl | |
| 653 | 1-Propyl | 2-(4-Hydroxyphenyl)ethyl | |
| 654 | 1-Propyl | 2-(4-Nitrophenyl)ethyl | |
| 655 | 1-Propyl | 3-Phenylpropyl | |
| 656 | 1-Propyl | 3-(4-Methylphenyl)propyl | |
| 657 | 1-Propyl | 3-(4-tert.-Butylphenyl)propyl | |
| 658 | 1-Propyl | 3-(2-Methoxyphenyl)propyl | |

| Nr. | $R^1$ | $R^2$ | Physik. Daten |
|---|---|---|---|
| 659 | 1-Propyl | 3-(4-Methoxyphenyl)propyl | |
| 660 | 1-Propyl | 3-(3,4-Dimethoxyphenyl)propyl | |
| 661 | 1-Propyl | 3-(4-tert.-Butoxyphenyl)propyl | |
| 662 | 1-Propyl | 3-(3-Trifluormethylphenyl)propyl | |
| 663 | 1-Propyl | 3-(4-Trifluormethoxyphenyl)propyl | |
| 664 | 1-Propyl | 3-(2-Fluorphenyl)propyl | |
| 665 | 1-Propyl | 3-(4-Fluorphenyl)propyl | |
| 666 | 1-Propyl | 3-(2-Chlorphenyl)propyl | |
| 667 | 1-Propyl | 3-(4-Chlorphenyl)propyl | |
| 668 | 1-Propyl | 3-(2,4-Dichlorphenyl)propyl | |
| 669 | 1-Propyl | 3-(2-Chlor-4-fluorphenyl)propyl | |
| 670 | 1-Propyl | 3-(4-Cyanophenyl)propyl | |
| 671 | 1-Propyl | 3-(4-Hydroxyphenyl)propyl | |
| 672 | 1-Propyl | 3-(4-Nitrophenyl)propyl | |
| 673 | 2-Propen-1-yl | Methyl | |
| 674 | 2-Propen-1-yl | Ethyl | |
| 675 | 2-Propen-1-yl | 1-Propyl | |
| 676 | 2-Propen-1-yl | 2-Propyl | |
| 677 | 2-Propen-1-yl | 1-Butyl | |
| 678 | 2-Propen-1-yl | 2-Butyl | |
| 679 | 2-Propen-1-yl | tert.-Butyl | |
| 680 | 2-Propen-1-yl | 2-Methyl-1-propyl | |
| 681 | 2-Propen-1-yl | 1-Pentyl | |
| 682 | 2-Propen-1-yl | 2-Methyl-1-butyl | |
| 683 | 2-Propen-1-yl | 2-Methyl-1-pentyl | |
| 684 | 2-Propen-1-yl | 2-Ethyl-1-butyl | |
| 685 | 2-Propen-1-yl | 2,2-Dimethyl-1-propyl | |

| Nr. | $R^1$ | $R^2$ | Physik. Daten |
|---|---|---|---|
| 686 | 2-Propen-1-yl | 3,3-Dimethyl-1-butyl | |
| 687 | 2-Propen-1-yl | 3-Methyl-1-butyl | |
| 688 | 2-Propen-1-yl | 1-Hexyl | |
| 689 | 2-Propen-1-yl | 2-Methyl-1-hexyl | |
| 690 | 2-Propen-1-yl | 2-Ethyl-1-hexyl | |
| 691 | 2-Propen-1-yl | 2,4,4-Trimethyl-1-pentyl | |
| 692 | 2-Propen-1-yl | 1-Heptyl | |
| 693 | 2-Propen-1-yl | 1-Octyl | |
| 694 | 2-Propen-1-yl | 1-Nonyl | |
| 695 | 2-Propen-1-yl | 1-Decyl | |
| 696 | 2-Propen-1-yl | 1-Undecyl | |
| 697 | 2-Propen-1-yl | 1-Dodecyl | |
| 698 | 2-Propen-1-yl | 2-Propen-1-yl | |
| 699 | 2-Propen-1-yl | 2-Buten-1-yl | |
| 700 | 2-Propen-1-yl | 2-Penten-1-yl | |
| 701 | 2-Propen-1-yl | 2-Hexen-1-yl | |
| 702 | 2-Propen-1-yl | 3-Methyl-2-buten-1-yl | |
| 703 | 2-Propen-1-yl | 2,3-Dimethyl-2-buten-1-yl | |
| 704 | 2-Propen-1-yl | 2-Hydroxyethyl | |
| 705 | 2-Propen-1-yl | 6-Hydroxy-1-hexyl | |
| 706 | 2-Propen-1-yl | 3-Chlor-1-butyl | |
| 707 | 2-Propen-1-yl | 4-Chlor-1-butyl | |
| 708 | 2-Propen-1-yl | 3-Chlor-2-methyl-1-propyl | |
| 709 | 2-Propen-1-yl | Cyclopropyl | |
| 710 | 2-Propen-1-yl | Cyclobutyl | |
| 711 | 2-Propen-1-yl | Cyclopentyl | |
| 712 | 2-Propen-1-yl | Cyclohexyl | |

| Nr. | $R^1$ | $R^2$ | Physik. Daten |
|---|---|---|---|
| 713 | 2-Propen-1-yl | Cycloheptyl | |
| 714 | 2-Propen-1-yl | Cyclooctyl | |
| 715 | 2-Propen-1-yl | Cyclodecyl | |
| 716 | 2-Propen-1-yl | Cyclododecyl | |
| 717 | 2-Propen-1-yl | 1-Methylcyclopropyl | |
| 718 | 2-Propen-1-yl | 1-Methylcyclopentyl | |
| 719 | 2-Propen-1-yl | 1-Methylcyclohexyl | |
| 720 | 2-Propen-1-yl | 2-Methylcyclohexyl | |
| 721 | 2-Propen-1-yl | 3-Methylcyclohexyl | |
| 722 | 2-Propen-1-yl | 4-Methylcyclohexyl | |
| 723 | 2-Propen-1-yl | 2,2-Dimethylcyclohexyl | |
| 724 | 2-Propen-1-yl | 3,3-Dimethylcyclohexyl | |
| 725 | 2-Propen-1-yl | 4,4-Dimethylcyclohexyl | |
| 726 | 2-Propen-1-yl | 2,6-Dimethylcyclohexyl | |
| 727 | 2-Propen-1-yl | 3,3,5-Trimethylcyclohexyl | |
| 728 | 2-Propen-1-yl | 3,3,5,5-Tetramethylcyclohexyl | |
| 729 | 2-Propen-1-yl | 4-Ethylcyclohexyl | |
| 730 | 2-Propen-1-yl | 4-Propylcyclohexyl | |
| 731 | 2-Propen-1-yl | 4-Isopropylcyclohexyl | |
| 732 | 2-Propen-1-yl | 4-tert.-Butylcyclohexyl | |
| 733 | 2-Propen-1-yl | cis-4-tert.-Butylcyclohexyl | |
| 734 | 2-Propen-1-yl | trans-4-tert.-Butylcyclohexyl | Harz (cis/trans = 2:3) |
| 735 | 2-Propen-1-yl | 4-tert.-Amylcyclohexyl | |
| 736 | 2-Propen-1-yl | Bicyclo[2.2.1]-hept-2-yl | |
| 737 | 2-Propen-1-yl | 1,7,7-Trimethylbicyclo[2.2.1]-hept-2-yl | |
| 738 | 2-Propen-1-yl | Bicyclo[4.4.0]dec-2-yl | |
| 739 | 2-Propen-1-yl | Bicyclo[4.4.0]dec-3-yl | |

| Nr. | $R^1$ | $R^2$ | Physik. Daten |
|---|---|---|---|
| 740 | 2-Propen-1-yl | 2,6,6-Trimethylbicyclo[3.1.1]-hept-3-yl | |
| 741 | 2-Propen-1-yl | Phenyl | |
| 742 | 2-Propen-1-yl | 2-Methylphenyl | |
| 743 | 2-Propen-1-yl | 3-Methylphenyl | |
| 744 | 2-Propen-1-yl | 4-Methylphenyl | |
| 745 | 2-Propen-1-yl | 2,4-Dimethylphenyl | |
| 746 | 2-Propen-1-yl | 4-Isopropylphenyl | |
| 747 | 2-Propen-1-yl | 4-tert.-Butylphenyl | |
| 748 | 2-Propen-1-yl | 2-Methoxyphenyl | |
| 749 | 2-Propen-1-yl | 4-Methoxyphenyl | |
| 750 | 2-Propen-1-yl | 3,4-Dimethoxyphenyl | |
| 751 | 2-Propen-1-yl | 4-tert.-Butoxyphenyl | |
| 752 | 2-Propen-1-yl | 2-Trifluormethylphenyl | |
| 753 | 2-Propen-1-yl | 3-Trifluormethylphenyl | |
| 754 | 2-Propen-1-yl | 4-Trifluormethylphenyl | |
| 755 | 2-Propen-1-yl | 4-Trifluormethoxyphenyl | |
| 756 | 2-Propen-1-yl | 2-Fluorphenyl | |
| 757 | 2-Propen-1-yl | 3-Fluorphenyl | |
| 758 | 2-Propen-1-yl | 4-Fluorphenyl | |
| 759 | 2-Propen-1-yl | 2,4-Difluorphenyl | |
| 760 | 2-Propen-1-yl | 2-Chlorphenyl | |
| 761 | 2-Propen-1-yl | 2-Chlor-4-fluorphenyl | |
| 762 | 2-Propen-1-yl | 3-Chlorphenyl | |
| 763 | 2-Propen-1-yl | 4-Chlorphenyl | |
| 764 | 2-Propen-1-yl | 2,4-Dichlorphenyl | |
| 765 | 2-Propen-1-yl | 2,6-Dichlorphenyl | |
| 766 | 2-Propen-1-yl | 3,5-Dichlorphenyl | |

| Nr. | $R^1$ | $R^2$ | Physik. Daten |
|---|---|---|---|
| 767 | 2-Propen-1-yl | 2-Cyanphenyl | |
| 768 | 2-Propen-1-yl | 3-Cyanphenyl | |
| 769 | 2-Propen-1-yl | 4-Cyanphenyl | |
| 770 | 2-Propen-1-yl | 2-Hydroxyphenyl | |
| 771 | 2-Propen-1-yl | 3-Hydroxyphenyl | |
| 772 | 2-Propen-1-yl | 4-Hydroxyphenyl | |
| 773 | 2-Propen-1-yl | 2-Nitrophenyl | |
| 774 | 2-Propen-1-yl | 3-Nitrophenyl | |
| 775 | 2-Propen-1-yl | 4-Nitrophenyl | |
| 776 | 2-Propen-1-yl | Benzyl | |
| 777 | 2-Propen-1-yl | 2-Methylbenzyl | |
| 778 | 2-Propen-1-yl | 4-Methylbenzyl | |
| 779 | 2-Propen-1-yl | 2,4-Dimethylbenzyl | |
| 780 | 2-Propen-1-yl | 4-Isopropylbenzyl | |
| 781 | 2-Propen-1-yl | 4-tert.-Butylbenzyl | |
| 782 | 2-Propen-1-yl | 2-Methoxybenzyl | |
| 783 | 2-Propen-1-yl | 3-Methoxybenzyl | |
| 784 | 2-Propen-1-yl | 4-Methoxybenzyl | |
| 785 | 2-Propen-1-yl | 3,4-Dimethoxybenzyl | |
| 786 | 2-Propen-1-yl | 4-tert.-Butoxybenzyl | |
| 787 | 2-Propen-1-yl | 2-Trifluormethylbenzyl | |
| 788 | 2-Propen-1-yl | 3-Trifluormethylbenzyl | |
| 789 | 2-Propen-1-yl | 4-Trifluormethylbenzyl | |
| 790 | 2-Propen-1-yl | 4-Trifluormethoxybenzyl | |
| 791 | 2-Propen-1-yl | 2-Fluorbenzyl | |
| 792 | 2-Propen-1-yl | 3-Fluorbenzyl | |
| 793 | 2-Propen-1-yl | 4-Fluorbenzyl | |

| Nr. | $R^1$ | $R^2$ | Physik. Daten |
|---|---|---|---|
| 794 | 2-Propen-1-yl | 2,4-Difluorbenzyl | |
| 795 | 2-Propen-1-yl | 2-Chlor-4-fluorbenzyl | |
| 796 | 2-Propen-1-yl | 2-Chlorbenzyl | |
| 797 | 2-Propen-1-yl | 3-Chlorbenzyl | |
| 798 | 2-Propen-1-yl | 4-Chlorbenzyl | |
| 799 | 2-Propen-1-yl | 2,4-Dichlorbenzyl | |
| 800 | 2-Propen-1-yl | 2,6-Dichlorbenzyl | |
| 801 | 2-Propen-1-yl | 3,5-Dichlorbenzyl | |
| 802 | 2-Propen-1-yl | 2-Cyanbenzyl | |
| 803 | 2-Propen-1-yl | 4-Cyanbenzyl | |
| 804 | 2-Propen-1-yl | 2-Hydroxybenzyl | |
| 805 | 2-Propen-1-yl | 3-Hydroxybenzyl | |
| 806 | 2-Propen-1-yl | 4-Hydroxybenzyl | |
| 807 | 2-Propen-1-yl | 2-Nitrobenzyl | |
| 808 | 2-Propen-1-yl | 3-Nitrobenzyl | |
| 809 | 2-Propen-1-yl | 4-Nitrobenzyl | |
| 810 | 2-Propen-1-yl | 2,4-Dinitrobenzyl | |
| 811 | 2-Propen-1-yl | 2-Phenylethyl | |
| 812 | 2-Propen-1-yl | 2-(4-Methylphenyl)ethyl | |
| 813 | 2-Propen-1-yl | 2-(4-tert.-Butylphenyl)ethyl | |
| 814 | 2-Propen-1-yl | 2-(2-Methoxyphenyl)ethyl | |
| 815 | 2-Propen-1-yl | 2-(4-Methoxyphenyl)ethyl | |
| 816 | 2-Propen-1-yl | 2-(3,4-Dimethoxyphenyl)ethyl | |
| 817 | 2-Propen-1-yl | 2-(4-tert.-Butoxyphenyl)ethyl | |
| 818 | 2-Propen-1-yl | 2-(4-Trifluormethoxyphenyl)ethyl | |
| 819 | 2-Propen-1-yl | 2-(3-Trifluormethylphenyl)ethyl | |
| 820 | 2-Propen-1-yl | 2-(2-Fluorphenyl)ethyl | |

| Nr. | $R^1$ | $R^2$ | Physik. Daten |
|---|---|---|---|
| 821 | 2-Propen-1-yl | 2-(4-Fluorphenyl)ethyl | |
| 822 | 2-Propen-1-yl | 2-(2-Chlorphenyl)ethyl | |
| 823 | 2-Propen-1-yl | 2-(4-Chlorphenyl)ethyl | |
| 824 | 2-Propen-1-yl | 2-(2,4-Dichlorphenyl)ethyl | |
| 825 | 2-Propen-1-yl | 2-(2-Chlor-4-fluorphenyl)ethyl | |
| 826 | 2-Propen-1-yl | 2-(4-Cyanophenyl)ethyl | |
| 827 | 2-Propen-1-yl | 2-(4-Hydroxyphenyl)ethyl | |
| 828 | 2-Propen-1-yl | 2-(4-Nitrophenyl)ethyl | |
| 829 | 2-Propen-1-yl | 3-Phenylpropyl | |
| 830 | 2-Propen-1-yl | 3-(4-Methylphenyl)propyl | |
| 831 | 2-Propen-1-yl | 3-(4-tert.-Butylphenyl)propyl | |
| 832 | 2-Propen-1-yl | 3-(2-Methoxyphenyl)propyl | |
| 833 | 2-Propen-1-yl | 3-(4-Methoxyphenyl)propyl | |
| 834 | 2-Propen-1-yl | 3-(3,4-Dimethoxyphenyl)propyl | |
| 835 | 2-Propen-1-yl | 3-(4-tert.-Butoxyphenyl)propyl | |
| 836 | 2-Propen-1-yl | 3-(3-Trifluormethylphenyl)propyl | |
| 837 | 2-Propen-1-yl | 3-(4-Trifluormethoxyphenyl)propyl | |
| 838 | 2-Propen-1-yl | 3-(2-Fluorphenyl)propyl | |
| 839 | 2-Propen-1-yl | 3-(4-Fluorphenyl)propyl | |
| 840 | 2-Propen-1-yl | 3-(2-Chlorphenyl)propyl | |
| 841 | 2-Propen-1-yl | 3-(4-Chlorphenyl)propyl | |
| 842 | 2-Propen-1-yl | 3-(2,4-Dichlorphenyl)propyl | |
| 843 | 2-Propen-1-yl | 3-(2-Chlor-4-fluorphenyl)propyl | |
| 844 | 2-Propen-1-yl | 3-(4-Cyanophenyl)propyl | |
| 845 | 2-Propen-1-yl | 3-(4-Hydroxyphenyl)propyl | |
| 846 | 2-Propen-1-yl | 3-(4-Nitrophenyl)propyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil Systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,

Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzubefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 20 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 62 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 5 werden in einer Mischung gelöst, die aus 80 Gew.-Teilen Xylol, 10 Gew.-Teilen des Anlagerungsproduktes von 8 bis 10 Mol Ethylenoxid an 1 Mol ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 15 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 39 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 40 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 50 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 59 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wur-

de, innig vermischt. Man erhält auf diese Weise eine Aufbereitung des Wirkstoffs mit guter Haftfähigkeit.

VIII. 40 Gew.-Teile der Verbindung Nr. 61 werden mit 10 Gew.-Teilen Natriumsalz eines Phensolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 67 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden 1-(4-(4-tert.-Butyl-phenyl)-cyclohexyl)-2,6-dimethylmorpholin (A) - bekannt aus EP 259 977 -, N,N-Dimethyl-4-(cyclohexylmethyl)-cyclohexylamin (B) und 4-(Cyclohexylmethyl)-cyclohexylamin (C) - beide bekannt aus US 3 981 766 - und trans-4-tert.-Butyl-N-benzylcyclohexylamin (D) - bekannt aus Journal of Organic Chemistry, 48 (1983), S. 3412-3422 - verwendet.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) inoculiert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzbefalls auf den Blattunterseiten.

Das Ergebnis zeigt, daß die Wirkstoffe 5, 15, 39, 40, 50, 59, 61, 62 und 67 bei der Anwendung als 0,025 %ige (Gew.%) Spritzbrühe eine bessere fungizide Wirkung zeigen (100 %) als die bekannten Vergleichswirkstoffe (A) und (C) (75 %).

Anwendungsbeispiel 2

Wirksamkeit gegen Botrytis cinerea

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten. Es wurde der Befall der Blätter festgestellt.

Das Ergebnis zeigt, daß die Wirkstoffe 5, 15, 59, 61, 62 und 67 bei der Anwendung als 0,05 %ige Spritzbrühe eine bessere fungizide Wirkung zeigen (90 %) als die bekannten Vergleichswirkstoffe (B), (C) und (D) (60 %).

## Patentansprüche

1. 4-(4-tert.-Butylphenyl)cyclohexylamine und ihre quaternären Ammoniumsalze der Formeln

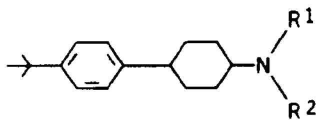

und

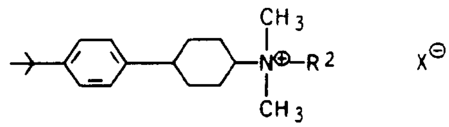

1 2

in denen

$R^1$

Wasserstoff, $C_1$-$C_6$-Alkyl, $C_3$-$C_6$-Alkenyl,

$R^2$

$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein-bis dreifach substituiertes Phenyl, unsubstituiertes oder ggf. ein- bis dreifach substituiertes Phenyl ($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können, mit der Maßgabe, daß nicht $R^1$ und $R^2$ gleichzeitig $C_1$-$C_4$-Alkyl bedeuten,

$X^{\ominus}$

ein pflanzenverträgliches Säureanion bedeutet,

und ihre pflanzenverträglichen Säureadditionssalze.

2. Verfahren zur Herstellung von 4-(4-tert.-Butylphenyl)cyclohexylaminen und ihren quaternären Ammoniumsalzen der Formeln 1 und 2, in denen

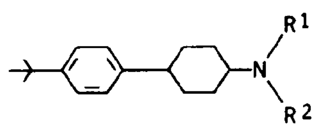

und

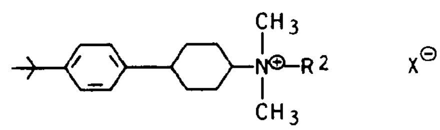

1 2

$R^1$

Wasserstoff, $C_1$-$C_6$-Alkyl, $C_3$-$C_6$-Alkenyl und

$R^2$

$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein-bis dreifach substituiertes Phenyl, unsubstituiertes oder ggf. ein- bis dreifach substituiertes Phenyl-($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können, mit der Maßgabe, daß nicht $R^1$ und $R^2$ gleichzeitig $C_1$-$C_4$-Alkyl bedeuten,

dadurch gekennzeichnet, daß man

a) ein Amin der Formel

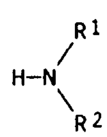

3

in der

$R^1$

Wasserstoff, $C_1$-$C_6$-Alkyl, $C_3$-$C_6$-Alkenyl und

$R^2$

$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein-bis dreifach substituiertes Phenyl, unsubstituiertes oder ggf. ein- bis dreifach substituiertes Phenyl($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogen-alkyl-,

$C_1$-$C_4$-Halogenalkoxy-, Halogen-, Cyan-, Hydroxy- oder Nitrogruppen sein können, mit der Maßgabe, daß nicht $R^1$ und $R^2$ gleichzeitig $C_1$-$C_4$-Alkyl bedeuten, mit 4-(4-tert.-Butylphenyl)cyclohexanon umsetzt und das entstehende Imin oder Enamin mit einem Reduktionsmittel reduziert, oder

b) eine Carbonylverbindung der Formel

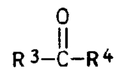

4

in der
$R^3$ und $R^4$ so beschaffen sind, daß der Rest

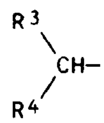

in seiner Gesamtheit dem Rest $R^2$ mit seinen oben genannten Bedeutungen entspricht, mit 4-(4-tert.-Butylphenyl)cyclohexylamin umsetzt und das entstandene Imin mit einem Reduktionsmittel reduziert und gegebenenfalls das so erhaltene Cyclohexylaminderivat der Formel 1 mit der Bedeutung $R^1$ = Wasserstoff mit einem Methylierungsmittel methyliert oder mit einer Säure in sein pflanzenverträgliches Säureadditionssalz überführt.

**3.** 4-(4-tert.-Butylphenyl)cyclohexanon.

**4.** cis-4-(4-tert.-Butylphenyl)cyclohexylamin.

**5.** trans-4-(4-tert.-Butylphenyl)cyclohexylamin.

**6.** Fungizid, dadurch gekennzeichnet, daß es einen inerten Trägerstoff und eine fungizid wirksame Menge eines 4-(4-tert.-Butylphenyl)cyclohexylamins oder seines quaternären Ammoniumsalzes der Formeln

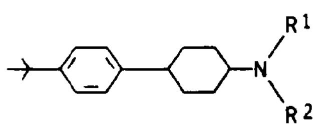

oder

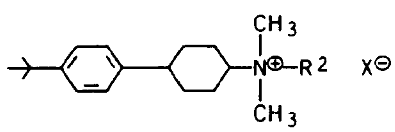

1 2

in denen
$R^1$
Wasserstoff, $C_1$-$C_6$-Alkyl, $C_3$-$C_6$-Alkenyl,
$R^2$
$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein-bis dreifach substituiertes Phenyl, unsubstituiertes oder ggf. ein- bis dreifach substituiertes Phenyl ($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können mit der Maßgabe, daß nicht $R^1$ und $R^2$ gleichzeitig $C_1$-$C_4$-Alkyl bedeuten, und
$X^{\ominus}$
ein pflanzenverträgliches Säureanion bedeutet,
oder seines pflanzenverträglichen Säureadditionssalzes enthält.

**7.** Verfahren zur Bekämpfung von Pilzen, dadurch gekennzeichnet, daß man die Pilze oder die vor Pilzbefall zu schützenden Pflanzen, Saatgüter, Materialien oder den Erdboden mit einer fungizid wirksamen Menge eines 4-(4-tert.-Butylphenyl)cyclohexylamins oder seines quartären Ammoniumsalzes der Formeln

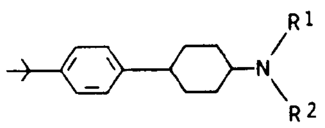

oder

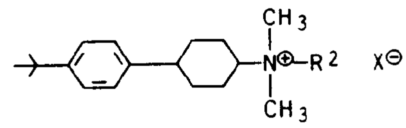

1 2

in denen

$R^1$

Wasserstoff, $C_1$-$C_6$-Alkyl, $C_3$-$C_6$-Alkenyl,

$R^2$

$C_1$-$C_{12}$-Alkyl, $C_1$-$C_{12}$-Halogenalkyl, $C_1$-$C_{12}$-Hydroxyalkyl, $C_3$-$C_{12}$-Cycloalkyl, $C_4$-$C_{12}$-Alkylcycloalkyl, $C_7$-$C_{12}$-Bicycloalkyl, $C_3$-$C_{12}$-Alkenyl, unsubstituiertes oder ggf. ein-bis dreifach substituiertes Phenyl, unsubstituiertes oder ggf. ein- bis dreifach substituiertes Phenyl ($C_1$-$C_3$)alkyl bedeuten, wobei die Substituenten jeweils gleich oder verschieden sind und $C_1$-$C_4$-Alkyl-, $C_1$-$C_4$-Alkoxy-, $C_1$-$C_4$-Halogenalkyl-, $C_1$-$C_4$-Halogenalkoxy-, Halogen-, Cyan-, Hydroxy- oder Nitrogruppen sein können mit der Maßgabe, daß nicht $R^1$ und $R^2$ gleichzeitig $C_1$-$C_4$-Alkyl bedeuten, und

$X^{\ominus}$

ein pflanzenverträgliches Säureanion bedeutet,

oder seines pflanzenverträglichen Säureadditionssalzes behandelt.

8. Verbindung der Formel 1 gemäß Anspruch 1, dadurch gekennzeichnet, daß $R^1$ Wasserstoff und $R^2$ 4-tert.-Butylcyclohexyl bedeutet.

9. Verbindung der Formel 2 gemäß Anspruch 1, dadurch gekennzeichnet, daß $R^2$ 4-tert.-Butylcyclohexyl und $X^{\ominus}$ Iodid bedeutet.

10. Verbindung der Formel 1 gemäß Anspruch 1, dadurch gekennzeichnet, daß $R^1$ Wasserstoff und $R^2$ Cyclohexyl bedeutet.

## Claims

1. A 4-(4-tert-butylphenyl)cyclohexylamine or a quaternary ammonium salt thereof of the formula

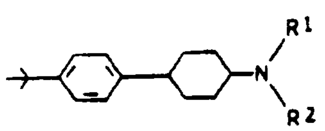

or

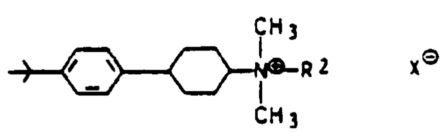

2

where

$R^1$ is hydrogen, $C_1$-$C_6$-alkyl or $C_3$-$C_6$-alkenyl,

$R^2$ is $C_1$-$C_{12}$-alkyl, $C_1$-$C_{12}$-haloalkyl, $C_1$-$C_{12}$-hydroxyalkyl, $C_3$-$C_{12}$-cycloalkyl, $C_4$-$C_{12}$-alkylcycloalkyl, $C_7$-$C_{12}$-bicycloalkyl, $C_3$-$C_{12}$-alkenyl, unsubstituted, monosubstituted, disubstituted or trisubstituted phenyl, or unsubstituted, monosubstituted, disubstituted or trisubstituted phenyl($C_1$-$C_3$)alkyl, possible substituents in each case being identical or different $C_1$-$C_4$-alkyl, $C_1$-$C_4$-alkoxy, $C_1$-$C_4$-haloalkyl, $C_1$-$C_4$-haloalkoxy, halogen, cyano, hydroxyl or nitro groups with the proviso that $R^1$ and $R^2$ are not simultaneously $C_1$-$C_4$-alkyl, and

$x^{\ominus}$ is a plant-tolerated acid anion, or a plant-tolerated acid addition salt thereof.

2. A process for the preparation of a 4-(4-tert-butylphenyl)cyclohexylamine or a quaternary ammonium salt thereof, of the formula 1 or 2

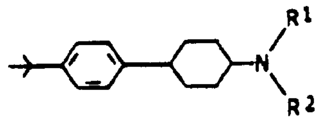

or

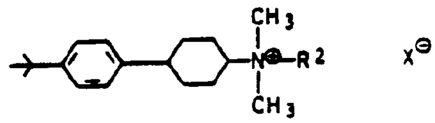

1 2

where
$R^1$ is hydrogen, $C_1$-$C_6$-alkyl or $C_3$-$C_6$-alkenyl and
$R^2$ is $C_1$-$C_{12}$-alkyl, $C_1$-$C_{12}$-haloalkyl, $C_1$-$C_{12}$-hydroxyalkyl, $C_3$-$C_{12}$-cycloalkyl, $C_4$-$C_{12}$-alkylcycloalkyl, $C_7$-$C_{12}$-bicycloalkyl, $C_3$-$C_{12}$-alkenyl, unsubstituted, monosubstituted, disubstituted or trisubstituted phenyl, or unsubstituted, monosubstituted, disubstituted or trisubstituted phenyl($C_1$-$C_3$)alkyl, possible substituents in each case being identical or different $C_1$-$C_4$-alkyl, $C_1$-$C_4$-alkoxy, $C_1$-$C_4$-haloalkyl, $C_1$-$C_4$-haloalkoxy, halogen, cyano, hydroxyl or nitro groups with the proviso that $R^1$ and $R^2$ are not simultaneously $C_1$-$C_4$-alkyl, which comprises

a) reacting an amine of the formula

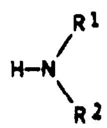

3

where
$R^1$ is hydrogen, $C_1$-$C_6$-alkyl or $C_3$-$C_6$-alkenyl and
$R^2$ is $C_1$-$C_{12}$-alkyl, $C_1$-$C_{12}$-haloalkyl, $C_1$-$C_{12}$-hydroxyalkyl, $C_3$-$C_{12}$-cycloalkyl, $C_4$-$C_{12}$-alkylcycloalkyl, $C_7$-$C_{12}$-bicycloalkyl, $C_3$-$C_{12}$-alkenyl, unsubstituted, monosubstituted, disubstituted or trisubstituted phenyl, or unsubstituted, monosubstituted, disubstituted or trisubstituted phenyl($C_1$-$C_3$)alkyl, possible substituents in each case being identical or different $C_1$-$C_4$-alkyl, $C_1$-$C_4$-alkoxy, $C_1$-$C_4$-haloalkyl, $C_1$-$C_4$-haloalkoxy, halogen, cyano, hydroxyl or nitro groups with the proviso that $R^1$ and $R^2$ are not simultaneously $C_1$-$C_4$-alkyl, with 4-(4-tert-butylphenyl)cyclohexanone, and reducing the resulting imine or enamine using a reducing agent, or

b) reacting a carbonyl compound of the formula

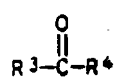

4

where $R^3$ and $R^4$ are such that the radical

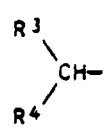

corresponds in its entirety to $R^2$ as defined above, with 4-(4-tert-butylphenyl)cyclohexylamine, and reducing the resulting imine using a reducing agent, and if desired methylating the resultant cyclohexylamine derivative of the formula I where $R^1$ is hydrogen using a methylating agent or converting it into its plant-tolerated acid addition salt using an acid.

**3.** 4-(4-tert-Butylphenyl)cyclohexanone.

**4.** cis-4-(4-tert-Butylphenyl)cyclohexylamine.

**5.** trans-4-(4-tert-Butylphenyl)cyclohexylamine.

**6.** A fungicide containing an inert carrier and a fungicidally effective amount of a 4-(4-tert-butylphenyl)cyclohexylamine or a quaternary ammonium salt thereof, of the formula

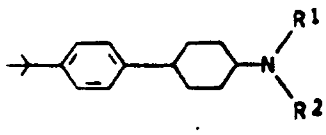

1

or

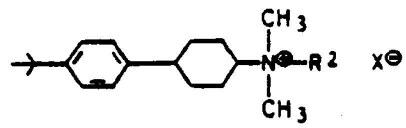

2

where

$R^1$ is hydrogen, $C_1$-$C_6$-alkyl or $C_3$-$C_6$-alkenyl,

$R^2$ is $C_1$-$C_{12}$-alkyl, $C_1$-$C_{12}$-haloalkyl, $C_1$-$C_{12}$-hydroxyalkyl, $C_3$-$C_{12}$-cycloalkyl, $C_4$-$C_{12}$-alkylcycloalkyl, $C_7$-$C_{12}$-bicycloalkyl, $C_3$-$C_{12}$-alkenyl, unsubstituted, monosubstituted, disubstituted or trisubstituted phenyl, or unsubstituted, monosubstituted, disubstituted or trisubstituted phenyl($C_1$-$C_3$)alkyl, possible substituents in each case being identical or different $C_1$-$C_4$-alkyl, $C_1$-$C_4$-alkoxy, $C_1$-$C_4$-haloalkyl, $C_1$-$C_4$-haloalkoxy, halogen, cyano, hydroxyl or nitro groups, with the proviso that $R^1$ and $R^2$ are not simultaneously $C_1$-$C_4$-alkyl, and

$X^{\ominus}$ is a plant-tolerated acid anion,

or a plant-tolerated acid addition salt thereof.

7. A method for controlling fungi, wherein the fungi or the plants, seed, materials or the soil to be protected against fungal attack are treated with a fungicidally effective amount of a 4-(4-tert-butylphenyl)cyclohexylamine or a quaternary ammonium salt thereof, of the formula

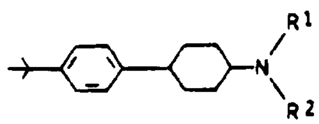

1

or

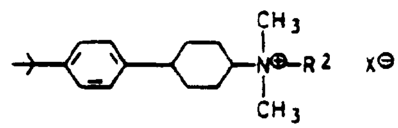

2

where

$R^1$ is hydrogen, $C_1$-$C_6$-alkyl or $C_3$-$C_6$-alkenyl,

$R^2$ is $C_1$-$C_{12}$-alkyl, $C_1$-$C_{12}$-haloalkyl, $C_1$-$C_{12}$-hydroxyalkyl, $C_3$-$C_{12}$-cycloalkyl, $C_4$-$C_{12}$-alkylcycloalkyl, $C_7$-$C_{12}$-bicycloalkyl, $C_3$-$C_{12}$-alkenyl, unsubstituted, monosubstituted, disubstituted or trisubstituted phenyl, or unsubstitututed, monosubstituted, disubstituted or trisubstituted phenyl($C_1$-$C_3$)alkyl, possible substituents in each case being identical or different $C_1$-$C_4$-alkyl, $C_1$-$C_4$-alkoxy, $C_1$-$C_4$-haloalkyl, $C_1$-$C_4$-haloalkoxy, halogen, cyano, hydroxyl or nitro groups with the proviso that $R^1$ and $R^2$ are not simultaneously $C_1$-$C_4$-alkyl, and

$X^{\ominus}$ is a plant-tolerated acid anion,

or a plant-tolerated acid addition salt thereof.

8. A compound of the formula 1 as claimed in claim 1, wherein $R^1$ is hydrogen and $R^2$ is 4-tert-butylcyclohexyl.

9. A compound of the formula 2 as claimed in claim 1, wherein $R^2$ is 4-tert-butylcyclohexyl and $X^{\ominus}$ is iodide.

10. A compound of the formula I as claimed in claim 1, wherein $R^1$ is hydrogen and $R^2$ is cyclohexyl.

## Revendications

1. 4-(4-tert-butylphényl) cyclohexylamine et ses sels d'ammonium quaternaire de formules

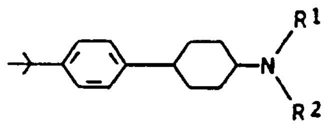 et 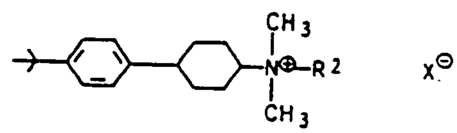

1 2

dans lesquelles
$R^1$ représente hydrogène, alkyle en C1-C6, alcényle en C3-C6,
$R^2$ représente alkyle en C1-C12, halogénalkyle en C1-C12, hydroxyalkyle en C1-C12, cycloalkyle en C3-C12, alkylcycloalkyle en C4-C12, bicycloalkyle en C7-C12, alcényle en C3-C12, phényle non substitué ou éventuellement substitué une à trois fois, phényle-alkyle en C1-C3 non substitué ou éventuellement substitué une à trois fois, les substituants étant identiques ou différents et pouvant être des groupes alkyle en C1-C4, alcoxy en C1-C4, halogénoalkyle en C1-C4, halogénalcoxy en C1-C4, halogène, cyano, hydroxy ou nitro, sous réserve que $R^1$ et $R^2$ ne soient pas simultanément alkyle en C1-C4 et
$X^{\ominus}$ représente un anion acide acceptable pour les plantes
et ses sels d'addition d'acide acceptables pour les plantes.

**2.** Procédé de préparation de 4-(4-tert-butylphényl) cyclohexylamine et ses sels d'amonium quaternaire des formules 1 et 2

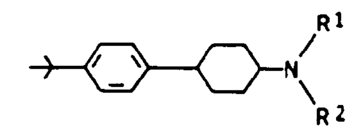 et 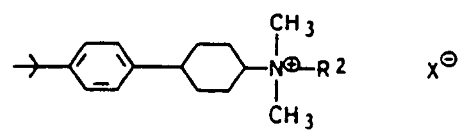

1 2

dans lesquelles
$R^1$ représente hydrogène, alkyle en C1-C6, alcényle en C3-C6, et
$R^2$ représente alkyle en C1-C12, halogénalkyle en C1-C12, hydroxyalkyle en C1-C12, cycloalkyle en C3-C12, alkylcycloalkyle en C4-C12, bicycloalkyle en C7-C12, alcényle en C3-C12, phényle non substitué ou éventuellement substitué une à trois fois, phényle-alkyle en C1-C3 non substitué ou éventuellement substitué une à trois fois, les substituants étant identiques ou différents et pouvant être des groupes alkyle en C1-C4, alcoxy en C1-C4, halogénoalkyle en C1-C4, halogénalcoxy en C1-C4, halogène, cyano, hydroxy ou nitro, sous réserve que $R^1$ et $R^2$ ne soient pas simultanément alkyle en C1-C4
caractérisé par le fait que

a) on fait réagir avec de la 4-(4-tert-butylphényl)cyclohexanone une amine de la formule

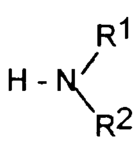 3

dans laquelle
$R^1$ représente hydrogène, alkyle en C1-C6, alcényle en C3-C6, et
R2 représente alkyle en C1-C12, halogénalkyle en C1-C12, hydroxyalkyle en C1-C12, cycloalkyle en C3-C12, alkylcycloalkyle en C4-C12, bicycloalkyle en C7-C12, alcényle en C3-C12, phényle non substitué ou éventuellement substitué une à trois fois, phényle-alkyle en C1-C3 non substitué ou éventuellement substitué une à trois fois, les substituants étant identiques ou différents et pouvant être des groupes alkyle en C1-C4, alcoxy en C1-C4, halogénoalkyle en C1-C4, halogénalcoxy en C1-C4, halogène, cyano, hydroxy ou nitro, sous réserve que $R^1$ et $R^2$ ne soient pas simultanément alkyle en C1-C4
et l'on réduit l'imine ou énamine obtenue avec un agent de réduction, ou

b) on fait réagir avec de la 4-(4-tert-butylphényl)-cyclohexylamine un composé carbonyle de la formule

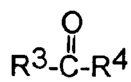

4

dans laquelle $R^3$ et $R^4$ sont choisis de manière que le reste

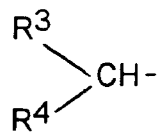

corresponde dans sa totalité au reste $R^2$ avec ses significations sus-indiquées, et l'imine obtenue est réduite avec un agent de réduction et le dérivé de cyclohexylamine de formule I ainsi obtenu avec la signification de $R^1$ = hydrogène est méthylé avec un agent de méthylation ou transformé avec un acide en son sel d'addition d'acide compatible avec les plantes.

**3.** 4-(4-tert-butylphényl)cyclohexanone

**4.** cis-4-(4-tert-butylphényl)cyclohexylamine

**5.** trans-4-(4-tert-butylphényl)cyclohexylamine

**6.** Fongicide caractérisé par le fait qu'il contient un matériau support inerte et une quantité efficace de point de vue fongicide d'une 4-(4-tert-butylphényl) cyclohexylamine ou son sel d'amonium quaternaire des formules

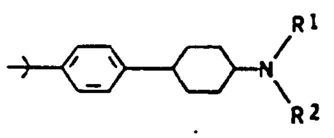

1

ou

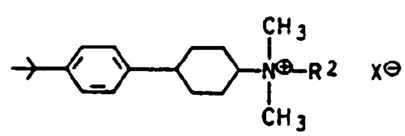

2

dans laquelle
$R^1$ représente hydrogène, alkyle en C1-C6, alcényle en C3-C6,
$R^2$ représente alkyle en C1-C12, halogénalkyle en C1-C12, hydroxyalkyle en C1-C12, cycloalkyle en C3-C12, alkylcycloalkyle en C4-C12, bicycloalkyle en C7-C12, alcényle en C3-C12, phényle non substitué ou éventuellement substitué une à trois fois, phényle-alkyle en C1-C3 non substitué ou éventuellement substitué une à trois fois, les substituants étant identiques ou différents et pouvant être des groupes alkyle en C1-C4, alcoxy en C1-C4, halogénoalkyle en C1-C4, halogénalcoxy en C1-C4, halogène, cyano, hydroxy ou nitro, sous réserve que $R^1$ et $R^2$ ne soient pas simultanément alkyle en C1-C4
et
$X^{\ominus}$ représente un anion acide acceptable pour les plantes et ses sels d'addition d'acide acceptables pour les plantes.

**7.** Procédé de lutte contre les champigons, caractérisé par le fait que l'on traite les champignons ou les plantes, semences, matériaux ou leur biotope à protéger contre l'attaque par champignons avec une quantité efficace au point de vue fongicide d'une 4-(4-tert-butylphényl)cyclohexylamine ou son sel d'amonium quaternaire des formules

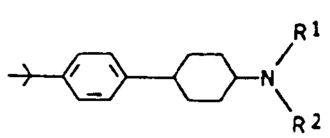

1

ou

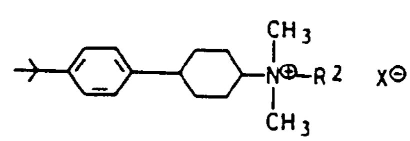

2

dans lesquelles
$R^1$ représente hydrogène, alkyle en C1-C6, alcényle en C3-C6,
$R^2$ représente alkyle en C1-C12, halogénalkyle en C1-C12, hydroxyalkyle en C1-C12, cycloalkyle en C3-C12, alkylcycloalkyle en C4-C12, bicycloalkyle en C7-C12, alcényle en C3-C12, phényle non substitué ou éventuellement substitué une à trois fois, phényle-alkyle en C1-C3 non substitué ou éventuellement substitué une à trois fois, les substituants étant identiques ou différents et pouvant être des groupes alkyle en C1-C4, alcoxy en C1-C4, halogénoalkyle en C1-C4, halogénalcoxy en C1-C4, halogène, cyano, hydroxy ou nitro, sous réserve que $R^1$ et $R^2$ ne soient pas simultanément alkyle en C1-C4 et
et
$X^{\ominus}$ représente un anion acide acceptable pour les plantes
et ses sels d'addition d'acide acceptables pour les plantes.

**8.** Composé de formule 1 selon la revendication 1, caractérisé par le fait que $R^1$ représente hydrogène et $R^2$ 4-tert-butylcyclohexyle.

**9.** Composé de formule 2 selon la revendication 1, caractérisé par le fait que $R^2$ représente 4-tert-butylcyclohexyle et $X^{\ominus}$ un iodure.

**10.** Composé de formule 1 selon la revendication 1, caractérisé par le fait que $R^1$ représente hydrogène et $R^2$ cyclohexyle.